# EUROPÄISCHE PATENTSCHRIFT

(11) **EP 0 824 533 B1**
(45) Veröffentlichungstag und Bekanntmachung des Hinweises auf die Patenterteilung: **14.11.2001**
(21) Anmeldenummer: 96913532.6
(22) Anmeldetag: 26.04.1996
(51) Int. Cl.: C07D 487/04, A01N 43/54

(54) **PYRAZOLO- 1,5a]-PYRIMIDINE, VERFAHREN ZU IHRER HERSTELLUNG UND IHRE VERWENDUNG**
PYRAZOLO-(1,5a)-PYRIMIDINES, PROCESS FOR PREPARING THE SAME AND THEIR USE
PYRAZOLO-(1,5a)-PYRIMIDINES, LEUR PROCEDE DE PREPARATION ET LEUR UTILISATION

(30) Priorität: 09.05.1995 DE 19516843; 20.01.1996 DE 19602072
(43) Veröffentlichungstag der Anmeldung: 25.02.1998
(73) Patentinhaber: BASF AKTIENGESELLSCHAFT, 67056 Ludwigshafen (DE)
(72) Erfinder: BENOIT, Remy, D-67435 Neustadt (DE); GROTE, Thomas, D-37077 Göttingen (DE); BAYER, Herbert, D-68159 Mannheim (DE); MÜLLER, Bernd, D-67227 Frankenthal (DE); OBERDORF, Klaus, D-69117 Heidelberg (DE); SAUTER, Hubert, D-68167 Mannheim (DE); AMMERMANN, Eberhard, D-64646 Heppenheim (DE); LORENZ, Gisela, D-67434 Hambach (DE); STRATHMANN, Siegfried, D-67117 Limburgerhof (DE)
(86) Internationale Anmeldenummer: EP9601774
(87) Internationale Veröffentlichungsnummer: WO9635690

(56) Entgegenhaltungen:
- EP-A- 0 254 426
- EP-A- 0 278 595
- EP-A- 0 398 692
- DE-A- 3 825 043

## Beschreibung

Die vorliegende Erfindung betrifft Pyrazolo-[1,5a]-pyrimidine der Formel I 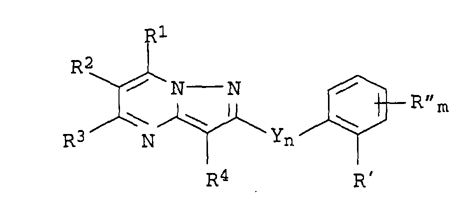 in der die Indizes und die Substituenten die folgende Bedeutung haben:
- n: 0 oder 1;
- Y: O, S, NR^{a}, OCH₂*, SCH₂*, NR^{a}CH₂*, CH₂O*, CH₂S*, CH₂NR^{a}*, CH₂CH₂, CR^{a}=CR^{b} oder C≡C, wobei die mit * gekennzeichneten Positionen die Bindung zum Phenylring kennzeichnen;
- R': H₃CO-CO-•=CHOCH₃, H₃CNH-CO-•=CHOCH₃, H₃CO-CO-•=NOCH₃, H₃CNH-CO-•=NOCH₃, H₂N-CO-•=NOCH₃, HO-CO-•=NOCH₃, H₃CO-CO-•=CHCH₃, H₃CO-CO-•=CHCH₂CH₃, H₃C-CO-•=COHCH₃, H₃C-CO-•=NOCH₃, H₃CCH₂-CO-•=NOCH₃, wobei • ein zum Phenylring gebundenes C-Atom darstellt;
N(OCH₃)-CO₂CH₃, N(CH₃)-CO₂CH₃, N(CH₂CH₃)-CO₂CH₃, oder
eine Gruppe R'.1-•=NOCH₃, R'.2-•=NOCH₃, R'.3-•=NOCH₃ oder R'.4-•=NOCH₃, 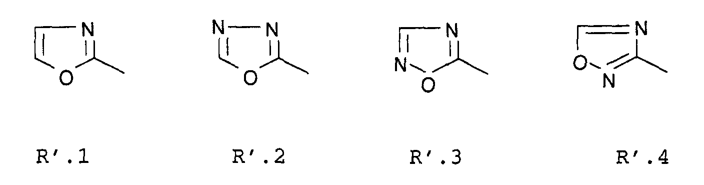
- m: 0, 1, 2 oder 3, wobei die Reste R" verschieden sein können, wenn m größer als 1 ist;
- R": Cyano, Halogen, C₁-C₄-Alkyl, C₁-C₄-Haloalkyl, C₁-C₄-Alkoxy, C₁-C₄-Haloalkoxy, C₁-C₄-Alkylthio oder C₁-C₄-Haloalkylthio;
- R¹,R³: Wasserstoff, Cyano, Nitro, Hydroxy, Amino, Halogen,
Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Phenyl, Phenyl-C₁-C₄-alkyl, Phenoxy, Phenyl-C₁-C₄-alkoxy, Phenylthio oder
NR^{c}R^{d}, NR^{c}NR^{d}R^{e}, CO-R^{c}, CO₂-R^{c}, CO-NR^{c}R^{d}, O-CO-R^{c}, O-CO₂-R^{c}, O-CO-NR^{c}R^{d}, NR^{c}-CO-R^{d}, NR^{c}-CO₂-R^{d} oder NR^{c}-CO-NR^{d}R^{e};
- R²: C₁-C₆-Wasserstoff, Halogen, C₁-C₆-Alkyl, C₁-C₆-Halogenalkyl, C₁-C₆-Alkoxy, C₁-C₆-Halogenalkoxy, Phenyl, Phenoxy, CO-R^{c}, CO₂R^{c} oder CR^{f}=NOR^{g};
- R⁴: Wasserstoff, Cyano, Nitro, Nitroso, Halogen,
C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Phenyl, Phenyl-C₁-C₄-alkyl, Phenoxy, Phenyl-C₁-C₄-alkoxy, Phenylthio, oder
NR^{c}R^{d}, CO-R^{c}, CO₂-R^{c} oder CR^{f}=NOR^{g};
- R^{a}, R^{b}: Wasserstoff, Halogen, C₁-C₄-Alkyl oder C₁-C₄-Halogenalkyl;
- R^{c}, R^{d}: und R^{e}
Wasserstoff, C₁-C₆-Alkyl, C₃-C₆-Alkenyl, C₃-C₆-Alkinyl, Phenyl oder Phenyl-C₁-C₄-alkyl;
- R^{f}: Wasserstoff, Cyano, Halogen,
C₁-C₆-Alkyl, C₁-C₆-Alkyloxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy, C₃-C₆-Alkinylthio, C₃-C₆-Cycloalkyl, C₃-C₆-Cycloalkyloxy, C₃-C₆-Cycloalkylthio, Phenyl, Phenoxy oder Phenylthio;
- R^{g}: Wasserstoff,
C₁-C₁₀-Alkyl, C₃-C₁₀-Alkenyl, C₃-C₁₀-Alkinyl, C₃-C₆-Cycloalkyl oder Phenyl,

Verfahren zu ihrer Herstellung, sie enthaltende Mittel und ihre Verwendung zur Bekämpfung von Schädlingen und Schadpilzen.

Aus der Literatur sind beispielsweise fungizid wirksame Verbindungen bekannt, welche anstelle des Pyrazolo-[1,5a]-pyrimidinrings einen Chinolinyl-, Isochinolinyl- oder Benzopyrazinylrest tragen (EP-A 278 595). Außerdem werden entsprechende Wirkstoffe mit Pyridyl-, Pyridazinyl-, Pyrimidyl-, Pyrazinyl-, Triazinyl- oder Tetrazinylresten beschrieben (EP-A398 692, EP-A 254 426).

Der vorliegenden Erfindung lagen neue Wirkstoffe mit verbesserten Wirkungsspektrum als Aufgabe zugrunde.

Demgemäß wurden die eingangs definierten Pyrazolo-[1,5a]-pyrimidine gefunden. Außerdem wurden Verfahren zu ihrer Herstellung, sie enthaltende Mittel und ihre Verwendung zur Bekämpfung von Schädlingen und Schadpilzen gefunden.

Die Herstellung der Verbindungen I erfolgt in Analogie zu verschiedenen, aus der Literatur an sich bekannten Methoden. Bei der Herstellung ist es im allgemeinen unerheblich, ob zunächst die Gruppierung R aufgebaut wird oder ob zunächst der Pyrazolopyrimidinrest an Y gekuppelt wird. Je nach der Stabilität der Zwischenprodukte ist es auch möglich auf den einzelnen Stufen des Aufbaus von R die Kupplung an das Pyrazolopyrimidin durchzuführen.

Demgemäß wird zur besseren Übersichtlichkeit in den folgenden Reaktionsgleichungen die Pyrazolopyrimidingruppierung oder ihre Vorstufe, wenn sie nicht an der Umsetzung beteiligt ist, verkürzt als R* dargestellt.

Bei der Herstellung der Verbindungen I, in denen Yₙ für OCH₂*, SCH₂* oder NR^{a}CH₂* [kollektiv als *"XCH*_{*2*}*"* bezeichnet] steht, geht man im allgemeinen so vor, daß man ein Benzylderivat der Formel IIa in an sich bekannter Weise in einem inerten organischen Lösungsmittel in Gegenwart einer Base mit einem Pyrazolopyrimidin der Formel IIIa umsetzt.

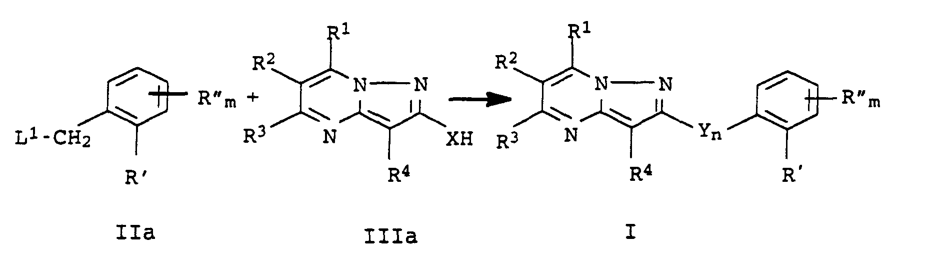

L¹ in der Formel IIa steht für eine nucleophil austauschbare Abgangsgruppe wie Halogen (z.B. Chlor, Brom und Jod) oder ein Sulfonat (z.B. Trifluormethylsulfonat, Phenylsulfonat und p-Methylphenylsulphonat).

Diese Umsetzung erfolgt üblicherweise bei Temperaturen von 0°C bis 120°C, vorzugsweise 15°C bis 60°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Methylenchlorid, Toluol, Aceton, Acetonitril und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxyd und Natriummethanolat.

Die Basen werden im allgemeinen im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IIIa in einem Über- oder Unterschuß bezogen auf IIa einzusetzen.

Die für die Herstellung der Verbindungen I benötigten Ausgangsstoffe der Formel IIa sind in der Literatur bekannt. Die entsprechenden Referate sind im Zusammenhang mit der Beschreibung der Herstellung der einzelnen Gruppen R' zitiert.

Die Ausgangsstoffe der Formel IIIa können in an sich bekannter Weise durch Umsetzung einer β-Dicarbonylverbindung IV mit einem 5-Aminopyrazol Va oder einem Cyanoessigsäurehydrazid Vb.

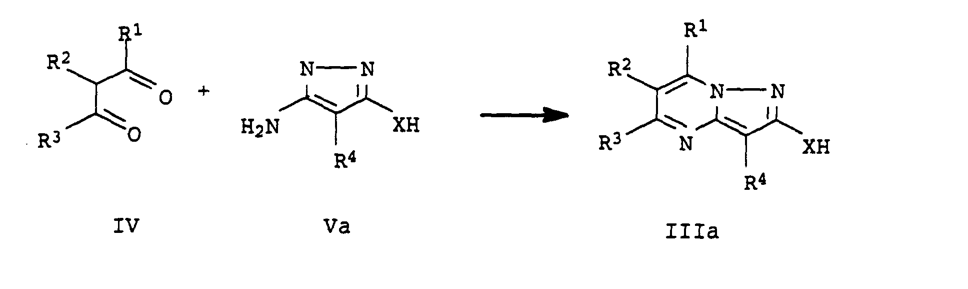

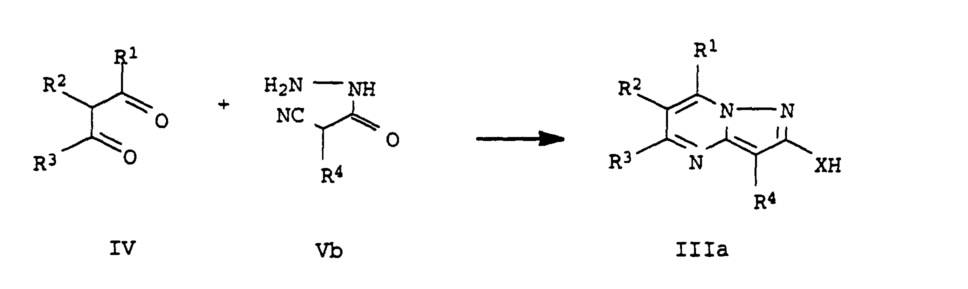

### Z.46:

Die Umsetzung der β-Dicarbonylverbindung IV mit dem 5-Aminopyrazol Va erfolgt in an sich bekannter Weise [Liebigs Ann. Chem. 647, 116 (1961)] in einem inerten Lösungsmittel in Gegenwart einer Säure oder eines Katalysators bei Temperaturen von 0°C bis 200°C, vorzugsweise 20°C bis 150°C, insbesondere 20°C bis 120°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid, Dimethylformamid und Wasser. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Trifluoressigsäure, Propionsäure, Oxalsäure und Zitronensäure Verwendung.

Die Säuren werden im allgemeinen in katalytischen Mengen eingesetzt, sie können aber auch äquimolar, im Überschuß oder als Lösungsmittel verwendet werden.

Die Edukte werden im allgemeinen in äquimolaren Mengen miteinander umgesetzt. Es kann für die Ausbeute vorteilhaft sein, IV in einem Über- oder Unterschuß bezogen auf V einzusetzen.

Die Umsetzung der β-Dicarbonylverbindung IV mit dem Cyanoessigsäurehydrazid Vb erfolgt in an sich bekannter Weise [Liebigs Ann. Chem. 647, 116 (1961)] in einem inerten Lösungsmittel entweder in Gegenwart einer Base oder einer Säure bei Temperaturen von 0°C bis 150°C, vorzugsweise 0°C bis 100°C, insbesondere 20°C bis 80°C.

Geeignete Lösungsmittel sind aliphatische Kohlenwasserstoffe wie Pentan, Hexan, Cyclohexan und Petrolether,aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Alkohole und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natrium- und Kaliumhydroxid. Die Basen werden im allgemeinen in katalytischen Mengen eingesetzt, sie können aber auch äquimolar, im Überschuß oder gegebenenfalls als Lösungsmittel verwendet werden.

Als Säuren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-IIIchlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden. Die Edukte werden im allgemeinen in äquimolaren Mengen miteinander umgesetzt. Es kann für die Ausbeute vorteilhaft sein, IV in einem Über- oder Unterschuß bezogen auf Vb einzusetzen.

Bei der Herstellung der Verbindungen I, in denen Yₙ für C≡C steht, geht man im allgemeinen so vor, daß man ein Phenylacetylen der Formel IIb in an sich bekannter Weise in einem inerten Lösungsmittel in Gegenwart eines Edelmatallkatalysators mit einem Pyrazolopyrimidinylhalogenid der Formel IIIb umsetzt.

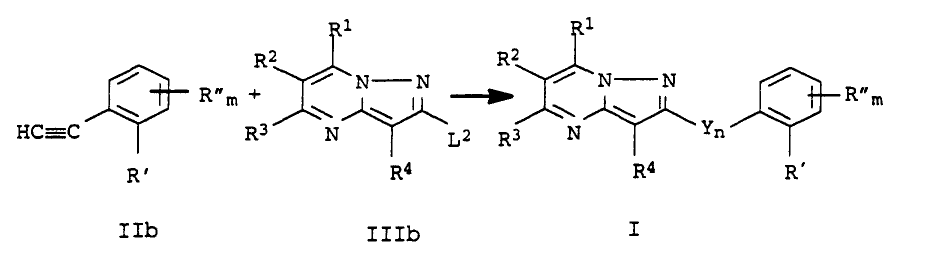

### Z in der Formel steht für

L² in der Formel IIIb steht für ein Halogenatom (z.B. Fluor, Chlor, Brom und Jod, vorzugsweise Brom und Jod).

Diese Umsetzung erfolgt üblicherweise bei Temperaturen von 0°C bis 200°C, vorzugsweise 15°C bis 160°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran. Es können auch Gemische der genannten Lösungsmittel verwendet werden.

Als Edelmetallkatalysatoren finden Pd(OCOCH₃)₂, PdCl₂, Pd[P(C₆H₅)₃]₄ und NiCl₂ Verwendung.

Die Edelmetallkatalysatoren werden im allgemeinen in Mengen von 0,1 mol-% bis 100 mol-%, vorzugsweise 5 mol-% bis 50 mol-%, insbesondere 10 mol-% bis 50 mol-%, bezogen auf die Verbindung IIb eingesetzt.

Die Edukte werden im allgemeinen in äquimolaren Mengen miteinander umgesetzt. Es kann für die Ausbeute vorteilhaft sein, IIb in einem Über- oder Unterschuß bezogen auf IIIb einzusetzen.

Die für diese Herstellung der Verbindungen I benötigten Ausgangsstoffe IIb sind in der Literatur bekannt (EP-A 582 925; DE-A 42 26 557) oder können gemäß der zitierten Literatur hergestellt werden.

Die Pyrazolopyrimidinylhalogenide der Formel IIIb sind ebenfalls bekannt oder können aus den entsprechenden Alkoholen der Formel IIIa (X = O) gemäß Liebigs Ann. Chem. 647, 116(1961) hergestellt werden.

Bei der Herstellung der Verbindungen I, in denen Yₙ für CH₂O*, CH₂S* oder CH₂NR^{a}* [kollektiv als *"CH*_{*2*}*X"* bezeichnet] steht, geht man im allgemeinen so vor, daß man ein entsprechendes Phenylderivat IIc in an sich bekannter Weise in einem inerten Lösungsmittel in Gegenwart einer Base mit einem Pyrazolopyrimidinylmethylenhalogenid der Formel IIIc umsetzt.

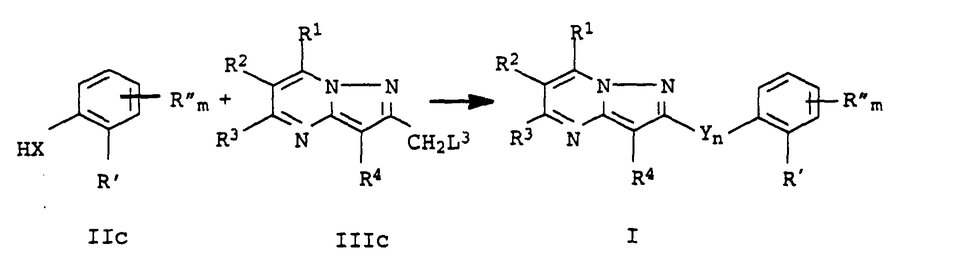

L³ in der Formel IIIc steht für eine nucleophil austauschbare Abgangsgruppe wie Halogen (z.B. Chlor, Brom und Jod) oder ein Sulfonat (z.B. Trifluormethylsulfonat, Phenylsulfonat und p-Methylphenylsulphonat).

Diese Umsetzung erfolgt üblicherweise bei Temperaturen von 0°C bis 200°C, vorzugsweise 15°C bis 150°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Aceton, Dimethylformamid und Dimethylsulfoxid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxid und Natriummethylat. Die Basen werden im allgemeinen im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IIc in einem Über- oder Unterschuß bezogen auf IIIc einzusetzen.

Die für diese Herstellung der Verbindungen I benötigten Ausgangsstoffe IIc sind in der Literatur bekannt (EP-A 260 794; GB-A 2 249 092) oder können gemäß der zitierten Literatur hergestellt werden.

Die Pyrazolopyrimidinylmethylenhalogenide der Formel IIIc sind ebenfalls bekannt oder können aus den entsprechenden Methylverbindungen durch Halogenierung mit elementarem Brom oder N-Brom-Succinimid gemäß bekannten Methoden [Farmaco. ed. Sci. 39, 888 (1984); J. Org. Chem. 53, 2055 (1988); Can. J. Chem. 60, 1233 (1982)] hergestellt werden.

Bei der Herstellung der Verbindungen I, in denen Yₙ für O, S oder NR⁵ [kollektiv als *"X"* bezeichnet] steht, geht man im allgemeinen so vor, daß man ein entsprechendes Phenylderivat der Formel IIc in an sich bekannter Weis ein einem inerten Lösungsmittel in Gegenwart einer Base mit einem Pyrazolopyrimidinylhalogenid der Formel IIIb kuppelt.

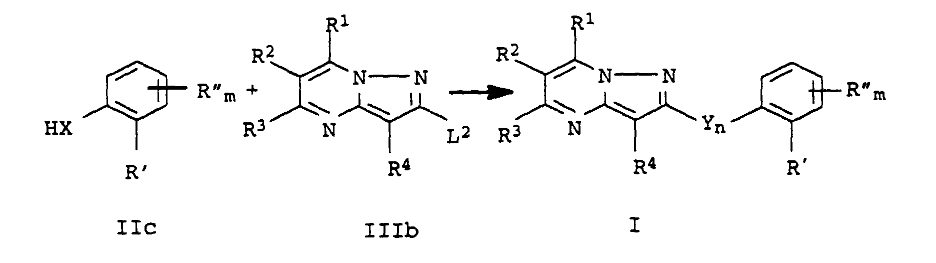

Die Kopplung erfolgt in einem inerten Lösungsmittel in Gegenwart einer Base sowie ggf. in Gegenwart eines Übergangsmetallkatalysators im Sinne einer Ullmann Kupplung [Russ. Chem. Rev. 43, 679 (1974); J. Org. Chem. 29, 977 (1964)] bzw. im Sinne einer nucleophilen Substitutionsreaktion [J. Chem. Soc. 1942, 381; J. Heterocycl. Chem. 15, 1513 (1978)].

Verbindungen I, in denen Yₙ für CR^{a}=CR^{b} steht, können außerdem dadurch erhalten werden, daß man eine Benzylphosphorverbindung der Formel IId in an sich bekannter Weise im Sinne einer Wittig-Umsetzung oder einer Horner-Wittig Reaktion in einem inerten organischen lösungsmittel in Gegenwart einer Base mit einer Methylpyrazolopyrimidin der Formel IIId umsetzt.

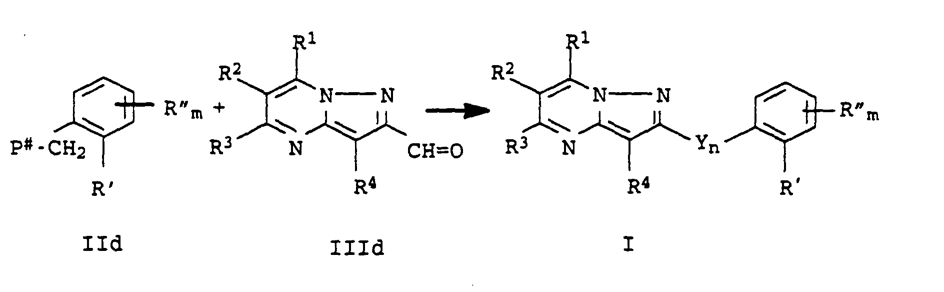

P# in der Formel IId steht für einen Triarylphosphonium Rest (z.B. Triphenylphosphonium Chlorid oder Triphenylphosphonium Bromid) oder eine Dialfylphosphinogruppe (z.B. Dimethylphosphino, Diethylphosphino und Di-isopropylphosphino).

Diese Umsetzung erfolgt üblicherweise bei Temperaturen von -30°C bis 60°C, vorzugsweise 0°C bis 40°C.
Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Diethylether, Tetrahydrofuran, Dimethylsulfoxid und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriummethanolat, Kalium-tert.-butanolat, Natriumhydrid, Kaliumcarbonat und Natriumhydrid. Die Basen werden im allgemeinen im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IId in einem Über- oder Unterschuß bezogen auf IIId einzusetzen.

Die für diese Herstellung der Verbindungen I benötigten Ausgangsstoffe IId sind in der Literatur bekannt oder können gemäß der zitierten Literatur hergestellt werden.

Verbindungen I, in denen Yₙ für CR^{a}=CR^{b} steht, können außerdem analog dem vorstehenden Verfahren dadurch erhalten werden, daß man ein Benzaldehyd der Formel IIe in an sich bekannter Weise im Sinne einer Wittig-Umsetzung oder einer Horner-Wittig Reaktion in einem inerten organischen lösungsmittel in Gegenwart einer Base mit einer Pyrazolopyrimidinphosphorverbindung der Formel IIIe umsetzt.

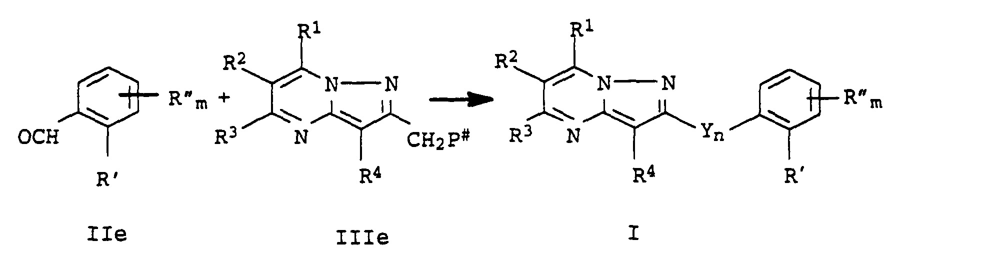

P# in der Formel IIIe steht für einen Triarylphosphonium Rest (z.B. Triphenylphosphonium Chlorid oder Triphenylphosphonium Bromid) oder eine Dialfylphosphinogruppe (z.B. Dimethylphosphino, Diethylphosphino und Di-isopropylphosphino).

Diese Umsetzung erfolgt üblicherweise bei Temperaturen von -30°C bis 60°C, vorzugsweise 0°C bis 40°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Diethylether, Tetrahydrofuran, Dimethylsulfoxid und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriummethanolat, Kalium-tert.-butanolat, Natriumhydrid und Kaliumcarbonat. Die Basen werden im allgemeinen im Überschuß oder gegebenenfalls als Lösungsmittel verwendet werden. Die Edukte werden im allgemeinen in äquimolaren Mengen miteinander umgesetzt.

Es kann für die Ausbeute vorteilhaft sein, IIe in einem Über- oder Unterschuß bezogen auf IIIe einzusetzen.

Die für diese Herstellung der Verbindungen I benötigten Ausgangsstoffe IIe sind in der Literatur bekannt (EP-A 499 823; EP-A 544 587; EP-A 621 277) oder können gemäß der zitierten Literatur hergestellt werden. Für die Herstellung der Verbindungen I benötigten Ausgangsstoffe IIIe können aus den entsprechenden Pyrazolopyrimidinylmethylhalogeniden der Formel IIIc durch Phosphorylierung gemäß bekannten Methoden [Synthesis 8, 743 (1992); Indian J. Chem. 22, 1050 (1983)] hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Verbindungen I, in denen Yₙ für OCH₂ steht erhält man besonders bevorzugt ausgehend von den entsprechenden Alkoholen der Formel IIa.1 und Phthaliden der Formel VI gemäß dem folgenden Reaktionsschema.

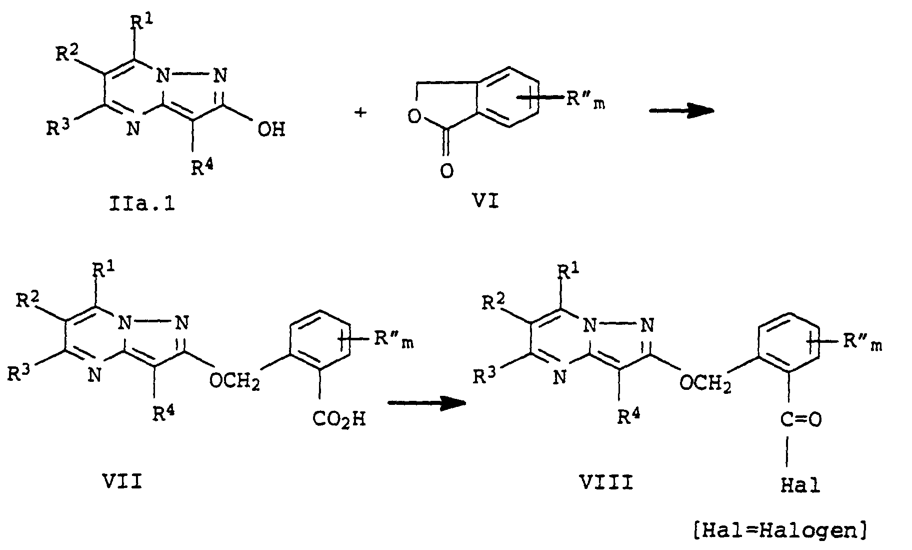

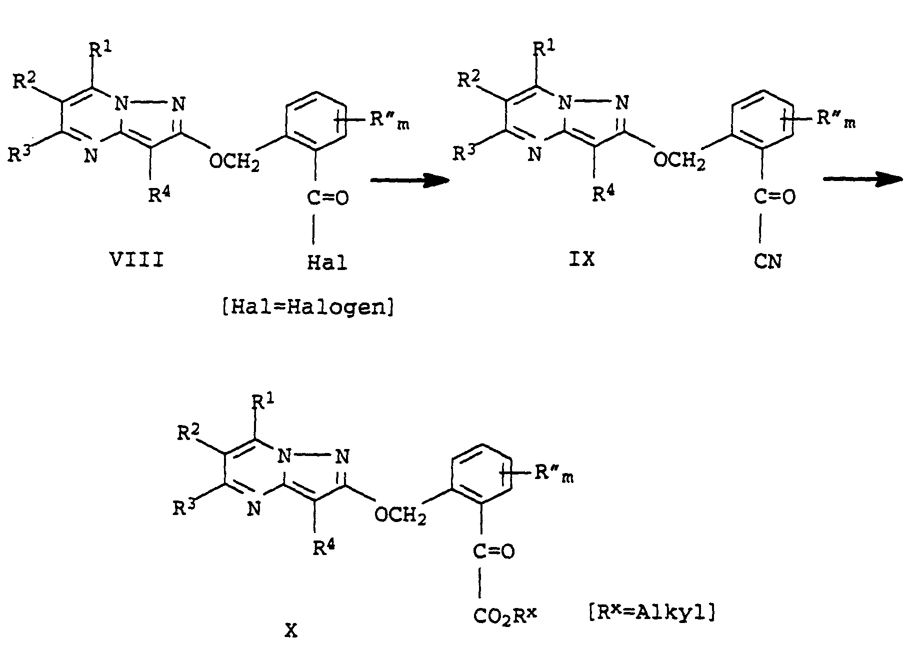

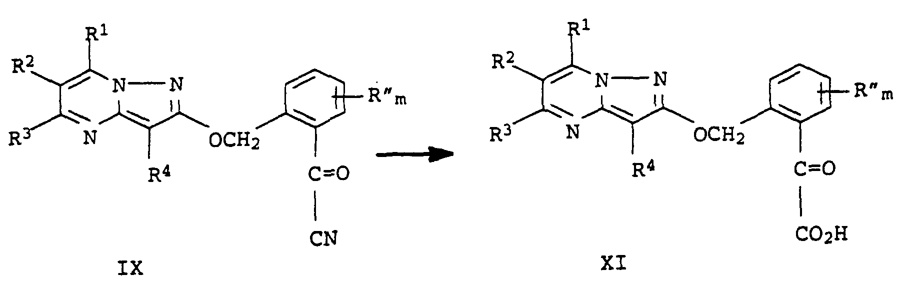

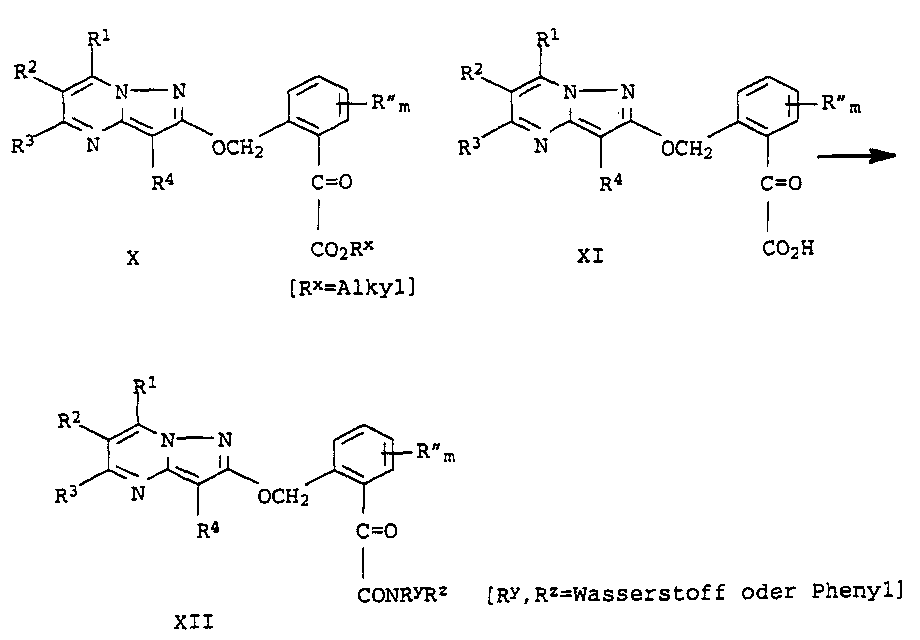

Die Umsetzung der Alkohole IIa.1 mit den Phthaliden VI erfolgt gemäß den in EP-A 493 711 beschriebenen Bedingungen. Die so erhaltenen Benzoesäuren VII können über die Zwischenstufen der Carbonsäurehalogenide VIII in die entsprechenden Cyanocarbonyle IX überführt werden.

Aus den Cyanocarbonylen IX erhält man einerseits im Wege der Pinner-Reaktion [Angew. Chem. 94, 1 (1982)] die entsprechenden α-Ketoester X oder durch Verseifung [J. Med. Chem. 20, 791 (1977) die entsprechenden α-Ketocarbonsäuren XI.

Sowohl X als auch XI können gemäß Synthesis 1978, 622 zu den Verbindungen XII amidiert werden.

Die α-Ketoester X bzw. die α-Ketoamide XII können nach bekannten Verfahren in die entsprechenden Verbindungen I überführt werden (EP-A 348 766, EP-A 178 826, DE-A 36 23 921, DE-A 37 05 389).

Der Aufbau der Gruppierung R' erfolgt grundsätzlich nach bekannten Methoden.

Verbindungen I, in denen *R' für H*_{*3*}*CO-CO-●=CHOCH*_{*3*} steht, erhält man beispielsweise nach den in EP-A 178 826, EP-A 226 917, EP-A 242 081 und in EP-A 278 595 beschriebenen Verfahren.

Verbindungen I, in denen *R' für H*_{*3*}*CO-CO-●=NOCH*_{*3*} *oder HO-CO-●=NOCH*_{*3*} steht, erhält man beispielsweise nach den in EP-A 253 213 und in EP-A 254 426 beschriebenen Verfahren.

Verbindungen I, in denen *R' für H*_{*3*}*CNH-CO-●=CHOCH*_{*3*}*, H*_{*3*}*CNH-CO-●=NOCH*_{*3*} *oder H*_{*2*}*N-CO-●=NOCH*_{*3*} steht, erhält man beispielsweise nach den in EP-A 398 692 und in EP-A 463 488 beschriebenen Verfahren.

Verbindungen I, in denen *R' für H*_{*3*}*CO-CO-●=CHCH*_{*3*} *oder H*_{*3*}*CO-CO-●=CHCH*_{*2*}*CH*_{*3*} steht, erhält man beispielsweise nach den in EP-A 513 580 beschriebenen Verfahren.

Verbindungen I, in denen *R' für H*_{*3*}*C-CO-●=NOCH*_{*3*} *oder H*_{*3*}*CCH*_{*2*}*-CO-●=NOCH*_{*3*} steht, erhält man beispielsweise nach den in EP-A 498 188 beschriebenen Verfahren.

Verbindungen I, in denen *R' für N(OCH*_{*3*}*)-CO*_{*2*}*CH*_{*3*}*, N(CH*_{*3*}*)-CO*_{*2*}*CH*_{*3*} *oder N(CH*_{*2*}*CH*_{*3*}*)-CO*_{*2*}*CH*_{*3*} steht, erhält man beispielsweise nach den in EP-A 498 396 und WO-A 93/15,046 beschriebenen Verfahren.

Verbindungen I, in denen *R' für CH(OCH*_{*3*}*)-CO*_{*2*}*CH*_{*3*} *oder CH(OCH*_{*3*}*)-CONHCH*_{*3*} steht, erhält man beispielsweise nach den in DE Anm. Nr. 44 32 336.0 beschriebenen Verfahren.

Verbindungen I, in denen *R' für eine Gruppe R'.1, R'.2, R'.3 oder R'.4* steht, erhält man beispielsweise nach den in WO-A 94/22,844 beschriebenen Verfahren.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl und 1-Ethyl-2-methylpropyl;
Halogenalkyl: geradkettige oder verzweigte Alkylgruppen mit 1 bis 4, 6 oder 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4, 6 oder 10 Kohlenstoffatomen (wie vorstehend genannt), welche über ein Sauerstoffatom (-O-) an das Gerüst gebunden sind;
Halogenalkoxy: geradkettige oder verzweigte Halogenalkylgruppen mit 1 bis 4, 6 oder 10 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 4, 6 oder 10 Kohlenstoffatomen (wie vorstehend genannt), welche über ein Schwefelatom (-S-) an das Gerüst gebunden sind;
Halogenalkylthio: geradkettige oder verzweigte Halogenalkylgruppen mit 1 bis 4, 6 oder 10 Kohlenstoffatomen (wie vorstehend genannt), welche über ein Schwefelatom (-S-) an das Gerüst gebunden sind;
Alkylamino: eine geradkettige oder verzweigte Alkylgruppe mit 1 bis 4 Kohlenstoffatomen (wie vorstehend genannt), welche über eine Aminogruppe (-NH-) an das Gerüst gebunden ist oder welches über eine Gruppe -NY¹- oder -NZ^{a}- an das Gerüst gebunden ist;
Dialkylamino: zwei voneinander unabhängige geradkettige oder verzweigte Alkylgruppen mit 1 bis 4 Kohlenstoffatomen (wie vorstehend genannt), welche über ein Stickstoffatom (-N:) an das Gerüst gebunden sind;
Alkylcarbonyl: eine geradkettige oder verzweigte Alkylgruppe mit 1 bis 4 Kohlenstoffatomen (wie vorstehend genannt), welche über eine Carbonylgruppe (-CO-) an das Gerüst gebunden ist;
Alkylcarbonyloxy: eine geradkettige oder verzweigte Alkylgruppe mit 1 bis 4 Kohlenstoffatomen (wie vorstehend genannt), welche über eine Carbonyloxygruppe (-CO₂-) an das Gerüst gebunden ist;
Alkylcarbonylamino: eine geradkettige oder verzweigte Alkylgruppe mit 1 bis 4 Kohlenstoffatomen (wie vorstehend genannt), welche über eine Carbonylaminogruppe (-CONH-) an das Gerüst gebunden ist;
Alkoxycarbonyl: eine geradkettige oder verzweigte Alkoxygruppe mit 1 bis 4 Kohlenstoffatomen (wie vorstehend genannt), welche über eine Carbonylgruppe (-CO-) an das Gerüst gebunden ist;
Halogenalkoxycarbonyl: eine geradkettige oder verzweigte Halogenalkoxygruppe mit 1 bis 4 Kohlenstoffatomen (wie vorstehend genannt), welche über eine Carbonylgruppe (-CO-) an das Gerüst gebunden ist;
Alkylthiocarbonyl: eine geradkettige oder verzweigte Alkylthigruppe mit 1 bis 4 Kohlenstoffatomen (wie vorstehend genannt), welche über eine Carbonylgruppe (-CO-) an das Gerüst gebunden ist;
Halogenalkylthiocarbonyl: eine geradkettige oder verzweigte Halogenalkylthigruppe mit 1 bis 4 Kohlenstoffatomen (wie vorstehend genannt), welche über eine Carbonylgruppe (-CO-) an das Gerüst gebunden ist;
Alkylaminocarbonyl: eine geradkettige oder verzweigte Alkylaminogruppe mit 1 bis 4 Kohlenstoffatomen (wie vorstehend genannt), welche über eine Carbonylgruppe (-CO-) an das Gerüst gebunden ist;
Dialkylaminocarbonyl: eine Dialkylaminogruppe (wie vorstehend genannt), welche über eine Carbonylgruppe (-CO-) an das Gerüst gebunden ist;
Alkoxycarbonyloxy: eine geradkettige oder verzweigte Alkoxygruppe mit 1 bis 4 Kohlenstoffatomen (wie vorstehend genannt), welche über eine Carbonyloxygruppe (-CO₂-) an das Gerüst gebunden ist;
Halogenalkoxycarbonyloxy: eine geradkettige oder verzweigte Halogenalkoxygruppe mit 1 bis 4 Kohlenstoffatomen (wie vorstehend genannt), welche über eine Carbonyloxygruppe (-CO₂-) an das Gerüst gebunden ist;
Alkylthiocarbonyloxy: eine geradkettige oder verzweigte Alkylthiogruppe mit 1 bis 4 Kohlenstoffatomen (wie vorstehend genannt), welche über eine Carbonyloxygruppe (-CO₂-) an das Gerüst gebunden ist;
Halogenalkylthiocarbonyloxy: eine geradkettige oder verzweigte Halogenalkylthiogruppe mit 1 bis 4 Kohlenstoffatomen (wie vorstehend genannt), welche über eine Carbonyloxygruppe (-CO₂-) an das Gerüst gebunden ist;
Alkylaminocarbonyloxy: eine geradkettige oder verzweigte Alkylaminogruppe mit 1 bis 4 Kohlenstoffatomen (wie vorstehend genannt), welche über eine Carbonyloxygruppe (-CO₂-) an das Gerüst gebunden ist;
Dialkylaminocarbonyloxy: eine Dialkylaminogruppe (wie vorstehend genannt), welche über eine Carbonyloxygruppe (-CO₂-) an das Gerüst gebunden ist;
Alkoxycarbonylamino: eine geradkettige oder verzweigte Alkoxygruppe mit 1 bis 4 Kohlenstoffatomen (wie vorstehend genannt), welche über eine Carbonylaminogruppe (-CONH-) an das Gerüst gebunden ist;
Halogenalkoxycarbonylamino: eine geradkettige oder verzweigte Halogenalkoxygruppe mit 1 bis 4 Kohlenstoffatomen (wie vorstehend genannt), welche über eine Carbonylaminogruppe (-CONH-) an das Gerüst gebunden ist;
Alkylthiocarbonylamino: eine geradkettige oder verzweigte Alkylthiogruppe mit 1 bis 4 Kohlenstoffatomen (wie vorstehend genannt), welche über eine Carbonylaminogruppe (-CONH-) an das Gerüst gebunden ist;
Halogenalkylthiocarbonylamino: eine geradkettige oder verzweigte Halogenalkylthiogruppe mit 1 bis 4 Kohlenstoffatomen (wie vorstehend genannt), welche über.eine Carbonylaminogruppe (-CONH-) an das Gerüst gebunden ist;
Alkylaminocarbonylamino: eine geradkettige oder verzweigte Alkylaminogruppe mit 1 bis 4 Kohlenstoffatomen (wie vorstehend genannt), welche über eine Carbonylaminogruppe (-CONH-) an das Gerüst gebunden ist;
Dialkylaminocarbonylamino: eine Dialkylaminogruppe (wie vorstehend genannt), welche über eine Carbonylaminogruppe (-CONH-) an das Gerüst gebunden ist;
Alkenyl: ungesättigte, geradkettige oder verzweigte Kohlenwasserstoffreste mit 2 oder 3 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-lpentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
Alkenyloxy: eine ungesättigte, geradkettige oder verzweigte Kohlenwasserstoffgruppe mit 2 oder 3 bis 6 oder 10 Kohlenstoffatomen und einer Doppelbindung in einer beliebigen Position (wie vorstehend genannt), welche über ein Sauerstoffatom (-O-) an das Gerüst gebunden ist;
Alkenylthio: eine ungesättigte, geradkettige oder verzweigte Kohlenwasserstoffgruppe mit 2 oder 3 bis 6 oder 10 Kohlenstoffatomen und einer Doppelbindung in einer beliebigen Position (wie vorstehend genannt), welche über ein Schwefelatom (-S-) an das Gerüst gebunden ist;
Alkinyl: geradkettige oder verzweigte Kohlenwasserstoffgruppen mit 2 oder 3 bis 6 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Alkinyloxy: eine geradkettige oder verzweigte Kohlenwasserstoffgruppe mit 2 oder 3 bis 6 oder 10 Kohlenstoffatomen und einer Dreifachbindung in einer beliebigen Position (wie vorstehend genannt), welche über ein Sauerstoffatom (-O-) an das Gerüst gebunden ist;
Alkinylthio: eine geradkettige oder verzweigte Kohlenwasserstoffgruppe mit 2 oder 3 bis 6 oder 10 Kohlenstoffatomen und einer Dreifachbindung in einer beliebigen Position (wie vorstehend genannt), welche über ein Schwefelatom (-S-) an das Gerüst gebunden ist;
Cycloalkyl: monocyclische Alkylgruppen mit 3 bis 6, 8 oder 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl; Cycloalkoxy: eine monocyclische Alkylgruppe mit 3 bis 6, 8 oder 12 Kohlenstoffringgliedern (wie vorstehend genannt), welche über ein Sauerstoffatom (-O-) an das Gerüst gebunden ist;
Cycloalkylthio: eine monocyclische Alkylgruppe mit 3 bis 6, 8 oder 12 Kohlenstoffringgliedern (wie vorstehend genannt), welche über ein Schwefelatom (-S-) an das Gerüst gebunden ist;
Heterocyclyl: ein gesättigter oder partiell ungesättigter cyclischer Rest, welcher neben Kohlenstoffatomen als Ringglieder Heteroatome aus der Gruppe Sauerstoff, Schwefel oder Stickstoff enthält: z.B. 5- oder 6-gliedrige Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl und 1,2,4-Tetrahydrotriazin-3-yl;
Heterocyclyloxy: ein Heterocyclylrest (wie vorstelend genannt), welcher über ein Sauerstoffatom (-O-) an das Gerüst gebunden ist;
Heterocyclylthio: ein Heterocyclylrest (wie vorstelend genannt), welcher über ein Schwefelatom (-S-) an das Gerüst gebunden ist;
Aryl: ein ein- bis dreikerniges aromatisches Ringsystem enthaltend 6 bis 14 Kohlenstoffringglieder, z.B. Phenyl, Naphthyl und Anthracenyl;
Arylalkyl: ein ein- bis dreikerniges aromatisches Ringsystem enthaltend 6 bis 14 Kohlenstoffringglieder (wie vorstehend genannt), welches über eine geradkettige oder verzweigte Alkylgruppe mit 1 bis 4, 6 oder 10 Kohlenstoffatomen an das Gerüst gebunden ist;
Aryloxy: ein ein- bis dreikerniges aromatisches Ringsystem enthaltend 6 bis 14 Kohlenstoffringglieder (wie vorstehend genannt), welches über ein Sauerstoffatom (-O-) an das Gerüst gebunden ist; Arylalkoxy: ein Arylalkylgruppe (wie vorstehend genannt), welche über ein Sauerstoffatom (-O-) an das Gerüst gebunden ist;
Arylthio: ein ein- bis dreikerniges aromatisches Ringsystem enthaltend 6 bis 14 Kohlenstoffringglieder (wie vorstehend genannt), welches über ein Schwefelatom (-S-) an das Gerüst gebunden ist;
Heteroaryl: ein aromatisches Ringsystem, welches neben Kohlenstoffringgliedern Heteroatome aus der Gruppe Sauerstoff, Schwefel und Stickstoff enthalten kann: Aryl wie vorstehend genannt oder ein- oder zweikerniges Heteroaryl, z.B.
   - 5-gliedriges Heteroaryl, enthaltend ein bis vier Stickstoffatome oder ein bis drei Stickstoffatome und ein Schwefeloder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
Heteroarylalkyl: ein Heteroarylrest (wie vorstehend genannt), welcher über eine geradkettige oder verzweigte Alkylgruppe mit 1 bis 4, 6 oder 10 Kohlenstoffatomen an das Gerüst gebunden ist;
Heteroaryloxy: ein Heteroarylrest (wie vorstehend genannt), welcher über ein Sauerstoffatom (-O-) an das Gerüst gebunden ist;
Heteroarylalkoxy: eine Heteroarylalkylgruppe (wie vorstehend genannt), welche über ein Sauerstoffatom (-O-) an das Gerüst gebunden ist;
Heteroarylthio: ein Heteroarylrest (wie vorstehend genannt), welcher über ein Schwefelatom (-S-) an das Gerüst gebunden ist;
Der Zusatz *"ggf. subst."* in Bezug auf *Alkyl-, Alkenyl- und Alkinylgruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom) ersetzt sein können und/ oder einen bis drei (vorzugsweise einen) der folgenden Reste tragen können:
   Cyano, Nitro, Hydroxy, Amino, Formyl, Carboxyl, Aminocarbonyl, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten;
   unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
   unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten.
Der Zusatz *"ggf. subst"* in Bezug auf die *cyclischen (gesättigten, ungesättigten oder aromatischen) Gruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom, insbesondere Fluor oder Chlor) ersetzt sein können und/oder einen bis vier (insbesondere einen bis drei) der folgenden Reste Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
und/oder einen bis drei (insbesondere einen) der folgenden Reste unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten, und oder einen oder zwei (insbesondere einen) der folgenden Reste Formyl oder CRⁱ=NORⁱⁱ, wobei Rⁱ Wasserstoff oder Alkyl und Rⁱⁱ Alkyl, Alkenyl, Alkinyl und Arylalkyl bedeutet und wobei die genannten Kohlenwasserstoffgruppen vorzugsweise 1 bzw. 3 bis 6 Kohlenstoffatome, insbesondere 1 bis 4 Kohlenstoffatome, enthalten und Aryl insbesondere Phenyl bedeutet, welches unsubstituiert ist oder durch übliche Gruppen substituiert sein kann, tragen kann oder bei denen zwei benachbarte C-Atome der cyclischen Systeme eine C₃-C₅-Alkylen-, C₃-C₅-Alkenylen-, Oxy-C₂-C₄-alkylen-, Oxy-C₁-C₃-alkylenoxy, Oxy-C₂-C₄-alkenylen-, Oxy-C₂-C₄-alkenylenoxy- oder Butadiendiylgruppe tragen können, wobei diese Brücken ihrerseits partiell oder vollständig halogeniert sein können und/oder einen bis drei, insbesondere einen oder zwei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio.

Unter üblichen Gruppen sind insbesondere die folgenden Substituenten zu verstehen: Halogen, Cyano, Nitro, C₁-C₄-Alkyl, C₁-C₄-Halogenalkyl, C₁-C₄-Alkyoxy, C₁-C₄-Halogenalkoxy, C₁-C₄-Alkylamino, Di-C₁-C₄-Alkylamino und C₁-C₄-Alkylthio.

Im Hinblick auf die biologische Wirkung werden besonders Pyrazolo-[1,5a]-pyrimidine der Formel I bevorzugt, in denen die Substituenten die folgende Bedeutung haben:
- R¹,R³: Wasserstoff, Cyano, Nitro, Hydroxy, Amino, Halogen,
C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Aryl, Aryl-C₁-C₄-alkyl, Aryloxy, Aryl-C₁-C₄-alkoxy, Arylthio, Heteroaryl, Heteroaryl-C₁-C₄-alkyl, Heteroaryloxy, Heteroaryl-C₁-C₄-alkoxy, Heteroarylthio, oder
NR^{c}R^{d}, NR^{c}NR^{d}R^{e}, CO-R^{c}, CO₂-R^{c}, CO-NR^{c}R^{d}, O-CO-R^{c}, O-CO₂-R^{c}, O-CO-NR^{c}R^{d}, NR^{c}-CO-R^{d}, NR^{c}-CO₂-R^{d} oder NR^{c}-CO-NR^{d}R^{e};
- R²: Wasserstoff, Halogen, C₁-C₆-Alkyl, C₁-C₆-Halogenalkyl, C₁-C₆-Alkoxy, C₁-C₆-Halogenalkoxy, Aryl, Aryloxy, Heteroaryl, Heteroaryloxy, CO-R^{c} CO₂R^{c} oder CR^{f}=NOR^{g};
- R⁴: Wasserstoff, Cyano, Nitro, Nitroso, Halogen,
C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Aryl, Aryl-C₁-C₄-alkyl, Aryloxy, Aryl-C₁-C₄-alkoxy, Arylthio, Heteroaryl, Heteroaryl-C₁-C₄-alkyl, Heteroaryloxy, Heteroaryl-C₁-C₄-alkoxy, Heteroarylthio, oder
NR^{c}R^{d}, CO-R^{c}, CO₂-R^{c} oder CR^{f}=NOR^{g};
- R^{c}, R^{d}: und R^{e} Wasserstoff, C₁-C₆-Alkyl, C₃-C₆-Alkenyl, C₃-C₆-Alkinyl, Aryl, Aryl-C₁-C₄-alkyl, Heteroaryl oder Heteroaryl-C₁-C₄-alkyl;
- R^{f}: Wasserstoff, Cyano, Halogen,
C₁-C₆-Alkyl, C₁-C₆-Alkoxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy oder C₃-C₆-Alkinylthio, wobei diese Gruppen partiell oder vollständig halogeniert sein können und einen bis drei der folgenden Reste tragen können: Cyano, Nitro, C₁-C₄-Alkoxy, C₁-C₄-Halogenalkoxy, C₁-C₄-Alkylthio, C₁-C₄-Halogenalkylthio, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragen können: Cyano, Nitro, C₁-C₄-Alkyl, C₁-C₄-Halogenalkyl, C₁-C₄-Alkoxy, C₁-C₄-Halogenalkoxyl C₁-C₄-Alkylthio, C₁-C₄-Halogenalkylthio, C₁-C₄-Alkylamino und Di-C₁-C₄-alkylamino;
C₃-C₆-Cycloalkyl, C₃-C₆-Cycloalkyloxy, C₃-C₆-Cycloalkylthio, Heterocyclyl, Heterocyclyloxy, Heterocyclylthio, Aryl, Aryloxy, Arylthio, Heteroaryl, Heteroaryloxy oder Heteroarylthio, wobei diese Gruppen partiell oder vollständig halogeniert sein können und einen bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino;
- R^{g}: Wasserstoff,
C₁-C₁₀-Alkyl, C₃-C₁₀-Alkenyl oder C₃-C₁₀-Alkinyl, wobei diese Gruppen partiell oder vollständig halogeniert sein können und einen bis drei der folgenden Reste tragen können: Cyano, Nitro, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkylcarbonyloxy, C₁-C₄-Alkylcarbonylamino, C₁-C₄-Alkoxycarbonyl, C₁-C₄-Halogenalkoxycarbonyl, C₁-C₄-Alkylthiocarbonyl, C₁-C₄-Halogenalkylthiocarbonyl, C₁-C₄-Alkylaminocarbonyl, Di-C₁-C₄-alkylaminocarbonyl, C₁-C₄-Alkoxycarbonyloxy, C₁-C₄-Halogenalkoxycarbonyloxy, C₁-C₄-Alkylthiocarbonyloxy, C₁-C₄-Halogenalkylthiocarbonyloxy, C₁-C₄-Alkylaminocarbonyloxy, Di-C₁-C₄-alkylaminocarbonyloxy, C₁-C₄-Alkoxycarbonylamino, C₁-C₄-Halogenalkoxycarbonylamino, C₁-C₄-Alkylthiocarbonylamino, C₁-C₄-Halogenalkylthiocarbonylamino, C₁-C₄-Alkylaminocarbonylamino, Di-C₁-C₄-alkylaminocarbonylamino, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragen können: Cyano, Nitro,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino;
C₃-C₆-Cycloalkyl, Heterocyclyl, Aryl oder Heteroaryl, wobei diese Gruppen partiell oder vollständig halogeniert sein können und einen bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkylcarbonyloxy, C₁-C₄-Alkylcarbonylamino, C₁-C₄-Alkoxycarbonyl, C₁-C₄-Halogenalkoxycarbonyl, C₁-C₄-Alkylthiocarbonyl, C₁-C₄-Halogenalkylthiocarbonyl, C₁-C₄-Alkylaminocarbonyl, Di-C₁-C₄-alkylaminocarbonyl, C₁-C₄-Alkoxycarbonyloxy, C₁-C₄-Halogenalkoxycarbonyloxy, C₁-C₄-Alkylthiocarbonyloxy, C₁-C₄-Halogenalkylthiocarbonyloxy, C₁-C₄-Alkylaminocarbonyloxy, Di-C₁-C₄-alkylaminocarbonyloxy, C₁-C₄-Alkoxycarbonylamino, C₁-C₄-Halogenalkoxycarbonylamino, C₁-C₄-Alkylthiocarbonylamino, C₁-C₄-Halogenalkylthiocarbonylamino, C₁-C₄-Alkylaminocarbonylamino, Di-C₁-C₄-alkylaminocarbonylamino, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragern können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino.

Im Hinblick auf ihre biologische Wirksamkeit sind daneben besonders Verbindungen der Formel I.A bevorzugt,

Im Hinblick auf ihre biologische Wirksamkeit sind daneben besonders Verbindungen der Formel I bevorzugt, in denen n für 1 steht und R' H₃CO-CO-•=CHOCH₃ bedeutet (I.A).

Insbesondere werden Verbindungen I.A bevorzugt, in denen R"ₘ für Wasserstoff, Halogen (besonders Chlor), C₁-C₂-Alkyl (besonders Methyl), C₁-C₂-Halogenalkyl (besonders Trifluormethyl) oder C₁-C₂-Alkoxy (besonders Methoxy) steht.

Insbesondere werden auch Verbindungen I.A bevorzugt, in denen Y für OCH₂, SCH₂, O, CH=CH, C=C oder CH₂O (besonders O, OCH₂, CH₂O, CH=CH und C≡C) steht.

Insbesondere werden auch Verbindungen I.A bevorzugt, in denen die Gruppen R¹, R², R³ und R⁴ für die folgenden Reste stehen: Wasserstoff, C₁-C₄-Alkyl, C₁-C₄-Halogenalkyl, C₁-C₄-Alkoxy, C₁-C₄-Alkoxycarbonyl und Phenyl.

Im Hinblick auf ihre biologische Wirksamkeit sind daneben besonders Verbindungen der Formel I bevorzugt, in denen n für 1 steht und R' H₃CO-CO-•=NOCH₃ bedeutet (I.B).

Insbesondere werden Verbindungen I.B bevorzugt, in denen R"ₘ für Wasserstoff, Halogen (besonders Chlor), C₁-C₂-Alkyl (besonders Methyl), C₁-C₂-Halogenalkyl (besonders Trifluormethyl) oder C₁-C₂-Alkoxy (besonders Methoxy) steht.

Insbesondere werden auch Verbindungen I.B bevorzugt, in denen Y für OCH₂, SCH₂, O, CH=CH, C≡C oder CH₂O (besonders O, OCH₂, CH₂O, CH=CH und C≡C) steht.

Insbesondere werden auch Verbindungen I.B bevorzugt, in denen die Gruppen R¹, R², R³ und R⁴ für die folgenden Reste stehen: Wasserstoff, C₁-C₄-Alkyl, C₁-C₄-Halogenalkyl, C₁-C₄-Alkoxy, C₁-C₄-Alkoxycarbonyl und Phenyl.

Im Hinblick auf ihre biologische Wirksamkeit sind daneben besonders Verbindungen der Formel I bevorzugt, in denen n für 1 steht und R' H₃CNH-CO-•=NOCH₃ bedeutet (I.C).

Insbesondere werden Verbindungen I.C bevorzugt, in denen R"ₘ für Wasserstoff, Halogen (besonders Chlor), C₁-C₂-Alkyl (besonders Methyl), C₁-C₂-Halogenalkyl (besonders Trifluormethyl) oder C₁-C₂-Alkoxy (besonders Methoxy) steht.

Insbesondere werden auch Verbindungen I.C bevorzugt, in denen Y für OCH₂, SCH₂, O, CH=CH, C≡C oder CH₂O (besonders O, OCH₂, CH₂O, CH=CH und C≡C) steht.

Insbesondere werden auch Verbindungen I.C bevorzugt, in denen die Gruppen R¹, R², R³ und R⁴ für die folgenden Reste stehen: Wasserstoff, C₁-C₄-Alkyl, C₁-C₄-Halogenalkyl, C₁-C₄-Alkoxy, C₁-C₄-Alkoxycarbonyl und Phenyl.

Im Hinblick auf ihre biologische Wirksamkeit sind daneben besonders Verbindungen der Formel I bevorzugt, in denen n für 1 steht und R' H₃CO-CO-•=CHCH₃ bedeutet (I.D).

Insbesondere werden Verbindungen I.D bevorzugt, in denen R"ₘ für Wasserstoff, Halogen (besonders Chlor), C₁-C₂-Alkyl (besonders Methyl), C₁-C₂-Halogenalkyl (besonders Trifluormethyl) oder C₁-C₂-Alkoxy (besonders Methoxy) steht.

Insbesondere werden auch Verbindungen I.D bevorzugt, in denen Y für OCH₂, SCH₂, O, CH=CH, C≡C oder CH₂O (besonders O, OCH₂, CH₂O, CH=CH und C≡C) steht.

Insbesondere werden auch Verbindungen I.D bevorzugt, in denen die Gruppen R¹, R², R³ und R⁴ für die folgenden Reste stehen: Wasserstoff, C₁-C₄-Alkyl, C₁-C₄-Halogenalkyl, C₁-C₄-Alkoxy, C₁-C₄-Alkoxycarbonyl und Phenyl.

Im Hinblick auf ihre biologische Wirksamkeit sind daneben besonders Verbindungen der Formel I bevorzugt, in denen n für 1 steht und R' N(OCH₃)-CO₂CH₃ bedeutet (I.E).

Insbesondere werden Verbindungen I.E bevorzugt, in denen R"ₘ für Wasserstoff, Halogen (besonders Chlor), C₁-C₂-Alkyl (besonders Methyl), C₁-C₂-Halogenalkyl (besonders Trifluormethyl) oder C₁-C₂-Alkoxy (besonders Methoxy) steht.

Insbesondere werden auch Verbindungen I.E bevorzugt, in denen Y für OCH₂, SCH₂, O, CH=CH, C≡C oder CH₂O (besonders O, OCH₂, CH₂O, CH=CH und C≡C) steht.

Insbesondere werden auch Verbindungen I.E bevorzugt, in denen die Gruppen R¹, R², R³ und R⁴ für die folgenden Reste stehen: Wasserstoff, C₁-C₄-Alkyl, C₁-C₄-Halogenalkyl, C₁-C₄-Alkoxy, C₁-C₄-Alkoxycarbonyl und Phenyl.

Die Verbindungen I können in bezug auf Doppelbindung der Gruppe Y und/oder der Gruppe R' in der "Z"- oder "E"-Konfiguration vorliegen. Demgemäß können bei der Herstellung der Verbindugnen I gemische der möglichen Isomere entstehen. Im allgemeinen sind solche Verbindungen I im Hinblick auf ihre biologische Wirksamkeit bevorzugt, in denen die Doppelbindung der Gruppe Y und/oder der Gruppe R' in der "E"-Konfiguration vorliegt.

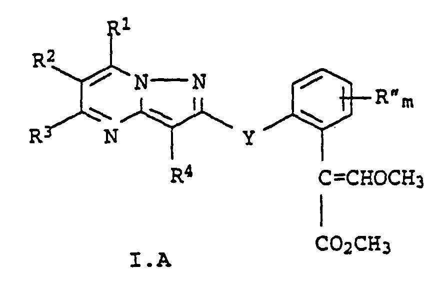

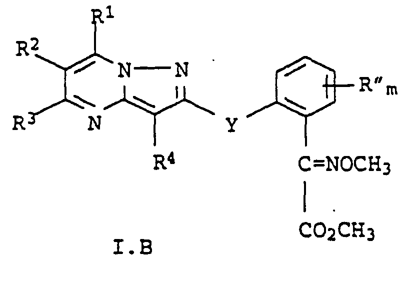

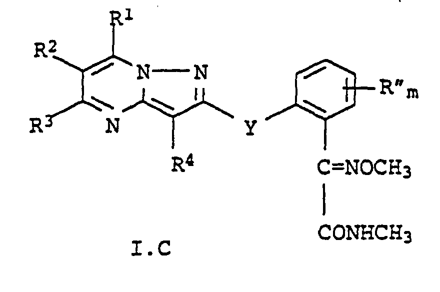

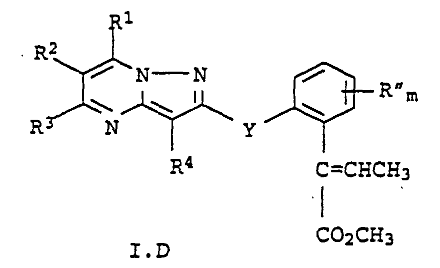

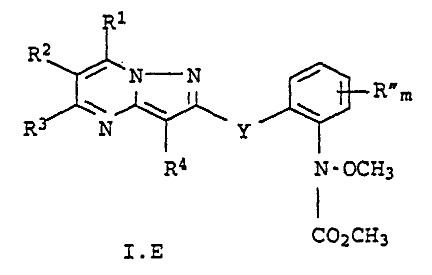

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungsformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
- I.: eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
- II.: eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
- III.: eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
- IV.: eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
- V.: eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
- VI.: eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
- VII.: eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
- VIII.: eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
- IX.: eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
- X.: eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol,2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-ylethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl (2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3, 5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-lH-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### Beispiel 1:

5,7-Dimethyl-2-hydroxypyrazolo[1,5a]pyrimidin

10 g (0,1 mol) Pentan-2,4-dion, 9,9 g (0,1 mol) 2-Cyanacethydrazid und 0,5 ml Eisessig werden in 20 ml Ethanol gelöst und 30 Minuten unter Rückfluß erhitzt. Nach Abkühlung auf Raumtemperatur wird die Suspension zu 120 ml 2n-Natronlauge gegeben und für 15 Minuten unter Rückfluß erhitzt. Die abgekühlte Reaktionsmischung wird mit 5 n Salzsäure auf pH-7 gestellt (Eiskühlung) und der entstehende Feststoff abgesaugt, mit Wasser und Pentan/Methyl tert.butylether: (1/5) gewaschen und getrocknet.

Man erhielt 11,1 g (68 % der Theorie) des gewünschten Produkts.

Fp = 233°C (Lit. 229-230°C) Liebigs Ann 1961, 647, 116.

¹H-NMR (ppm): 2,40 (s, 3H); 2,55 (s, 3H); 5,70 (s, 1H) 6,65 (s, 1H); 10,90 (m,1H)

### Beispiel 2:

2-[2-(5,7-Dimethyl-pyrazolo[1,5a]pyrimidin-2-yloxymethyl)-phenyl]-3-methoxy-acrylsäuremethylester

1,25 g (7,7 mmol) 5,7-Dimethyl-2-hydroxypyrazolo[1,5a]pyrimidin werden in 30 ml Methanol gelöst und mit 1,4 g 30 % Natriummethanolatlösung in Methanol (1,1 eq) versetzt. Nach 10 minütigem Rühren bei Raumtemperatur wird die Lösung im Vakuum eingedampft und der Rückstand in 30 ml Dimethylformamid aufgenommen.

Zu dieser Lösung werden 0,12 g (0,1 eq) Kaliumjodid und 2,0 g (7,0 mmol) 2-(2-Bromomethyl-phenyl)methoxy-iminoessigsäuremethylester gelöst in 20 ml DMF zugetropft. Nach vierstündigem Rühren bei Raumtempertur wird die Reaktionsmischung auf Eis gegeben und der entstehende Niederschlag abgesaugt und mit Pentan nachgewaschen. Man erhält 1,7 g der Titelverbindung mit einem Schmelzpunkt Fp = 136-139°C. Ausbeute: 66 % der Theorie

¹H-NMR: 2,50 (s,3H); 2,63 (s,3H); 3,69 (s,3H); 3,83 (s,3H); 5,22 (s,2H); 5,88 (s,1H); 6,41 (s,1H); 7,25 (m,1H); 7,33 (m,2H); 7,60 (s,1H) 7,62 (m,1H).

### Beispiel 3:

2-[2-(5,7-Dimethyl-pyrazolo[1,5a]pyrimidin-2-yloxymethyl)-phenyl]-methoxy-iminoessigsäuremethylester

3,13 g (19,2 mmol) 5,7-Dimethyl-2-hydroxypyrazolo[1,5a]pyrimidin werden in 50 ml Methanol gelöst und mit 3,5 g 30% Natriummethanolatlösung in Methanol (1,1 eq) versetzt. Nach 10minütigem Rühren bei Raumtemperatur wird die Lösung im Vakuum eingedampft und der Rückstand in 50 ml Dimethylformamid aufgenommen.

Zu dieser Lösung werden 0,3 g (1,1 eq) Kaliumjodid und 5,0 g (17,5 mmol) 2-(2-Bromomethyl-phenyl)methoxy-iminoessigsäuremethylester gelöst in 30 ml DMF zugetropft. Nach vierstündigem Rühren bei Raumtempertur wird die Reaktionsmischung auf Eis gegeben und der entstehende Niederschlag abgesaugt und mit Pentan nachgewaschen. Man erhält 4,8 g der Titelverbindung mit einem Schmelzpunkt Fp = 123-126°C. Ausbeute: 75 % der Theorie

¹H-NMR: 2,30 (s,3H); 2,43 (s,3H); 3,86 (s,3H); 4,06 (s,3H); 5,24 (s,2H); 5,88 (s,1H); 6,42 (s,1H); 7,20 (m,1H); 7,42 (m,2H); 7,64 (s,1H).

### Beispiel 4:

2-[2-(5,7-Dimethyl-pyrazolo[1,5a]pyrimidin-2-yloxymethyl)-phenyl]-methoxy-iminoessigsäuremethylester

2,5 g (6,8 mmol) 2[2-(5,7-Dimethyl-pyrazolo[1,5a]pyrimidin-2-yx-loxymethyl)-phenyl]methoxy-iminoessigsäuremethylester (Produkt aus Beispiel 3) werden in 20 ml Tetrahydrofuran gelöst, 11,6 g 40%ige Methylaminlösung (25 eq) zugesetzt und die Reaktionsmischung bei Raumtemperatur für 12 Stunden gerührt.

Nach Eindampfen der Reaktionslösung im Vakuum wird der Rückstand in Essigester aufgenommen und zweimal mit Wasser gewaschen. Die organische Phase wird über Natriumsulfat getrocknet und im Vakuum eingeengt. Man erhält 2,3 g bräunliche Kristalle mit einem Schmelzpunkt Fp = 135-138°C. Ausbeute: 95 % der Theorie

¹H-NMR: 2,48 (s,3H); 2,61 (s,3H); 2,93 (d,3H); 3,96 (s,3H); 5,22 (s,2H); 5, 88 (s,1H); 6,40 (s,1H); 6,82 (m,1H); 7,25 (m,1H); 7,40 (m,2H); 7,60 (m,1H).

### Beispiel 5:

2-[2-(5,7-Dimethyl-pyrazolo[1,5a]pyrimidin-2-yloxymethyl)-phenyl]-crotonsäuremethylester

2,3 g (14 mmol) 5,7-Dimethyl-2-hydroxypyrazolo[1,5a]pyrimidin werden in 30 ml Methanol gelöst und mit 2,5 g 30 %iger Natriummethanolatlösung in Methanol (1,1 eq) versetzt. Nach 15minütigem Rühren bei Raumtemperatur wird die Lösung im Vakuum eingedampft und der Rückstand in 50 ml Dimethylformamid aufgenommen.

Zu dieser Lösung werden 0,2 g (1,1 eq) Kaliumjodid und 3,2 g (12 mmol) 2-(2-Bromomethyl-phenyl)crotonsäuremethylester gelöst in 30 ml DMF zugetropft. Nach eintägigem Rühren bei Raumtemperatur wird die Reaktionsmischung auf Eis gegeben und der entstehende Niederschlag abgesaugt und mit Penton nachgewaschen. Man erhält 3,47 g der Titelverbindung mit einem Schmelzpunkt Fp = 109°-110°C. Ausbeute: 83 % d. Theorie.

¹H-NMR: 1,70 (d,3H); 2,52 (s,3H); 2,68 (s,3H); 3,72 (s,3H); 5,20 (s,2H); 5,90 (s,1H); 6,43 (s,1H); 7,21 (m,1H); 7,30 (q,1H); 7,40 (m,2H); 7,65 (m,1H).

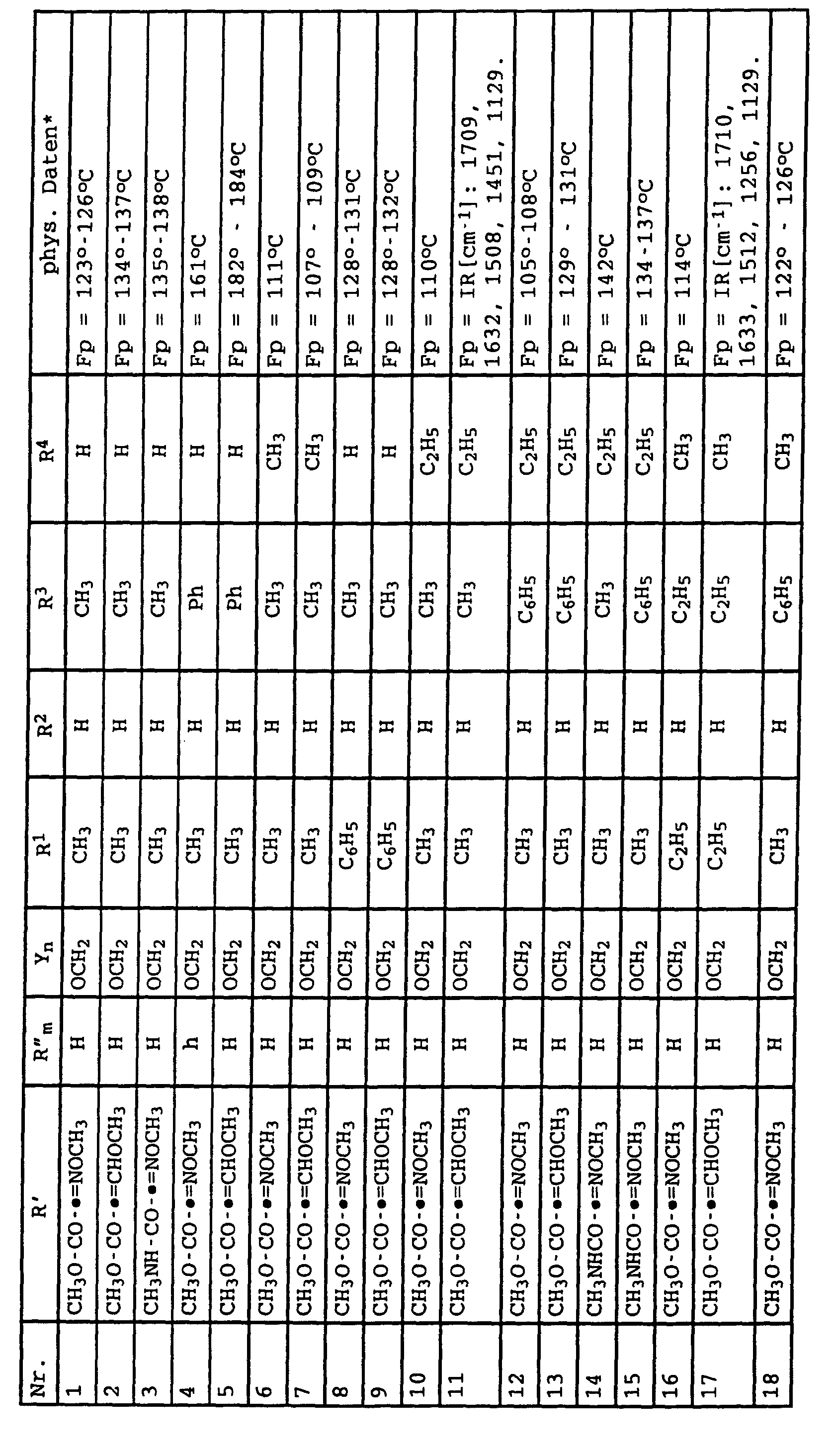

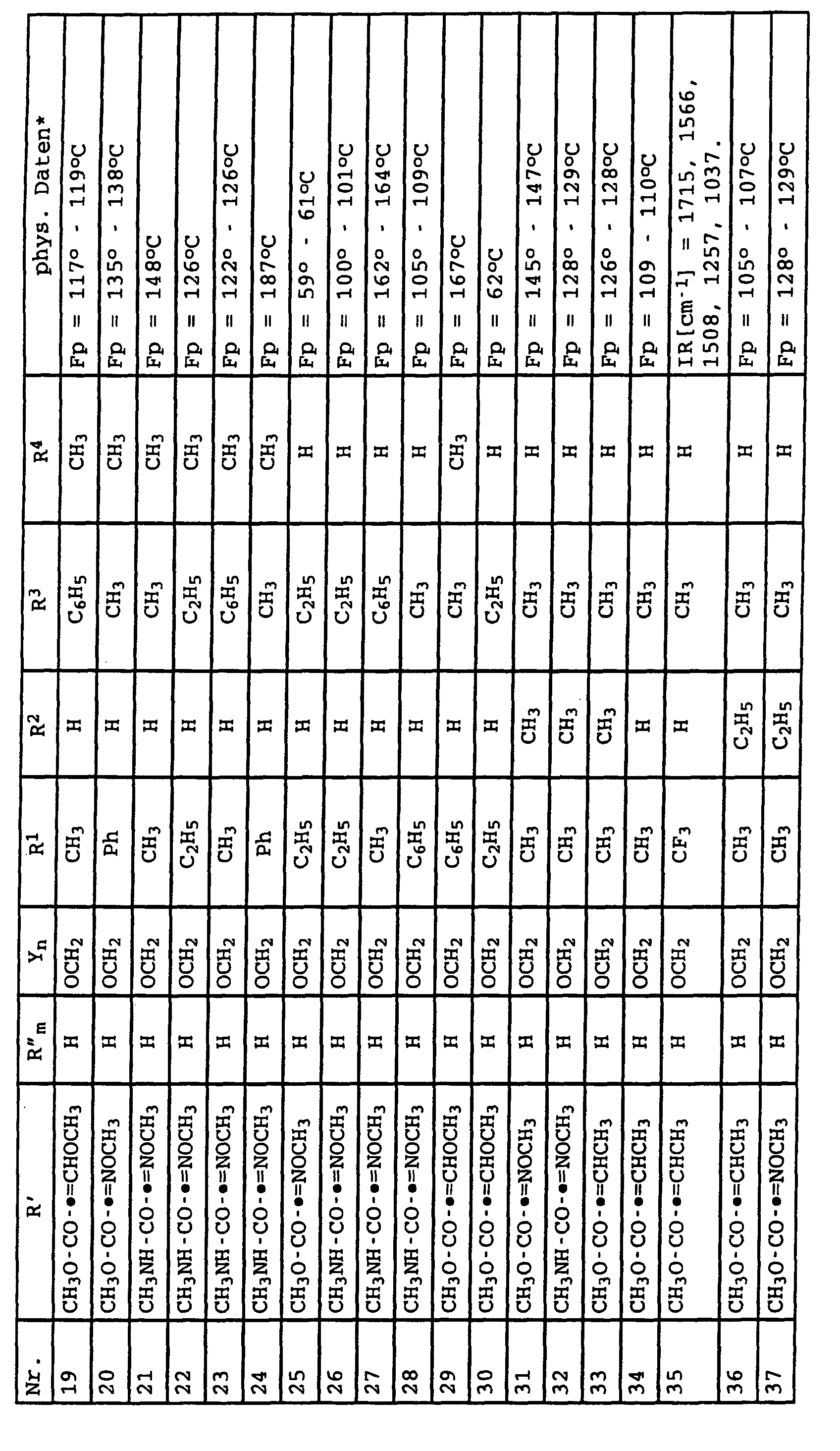

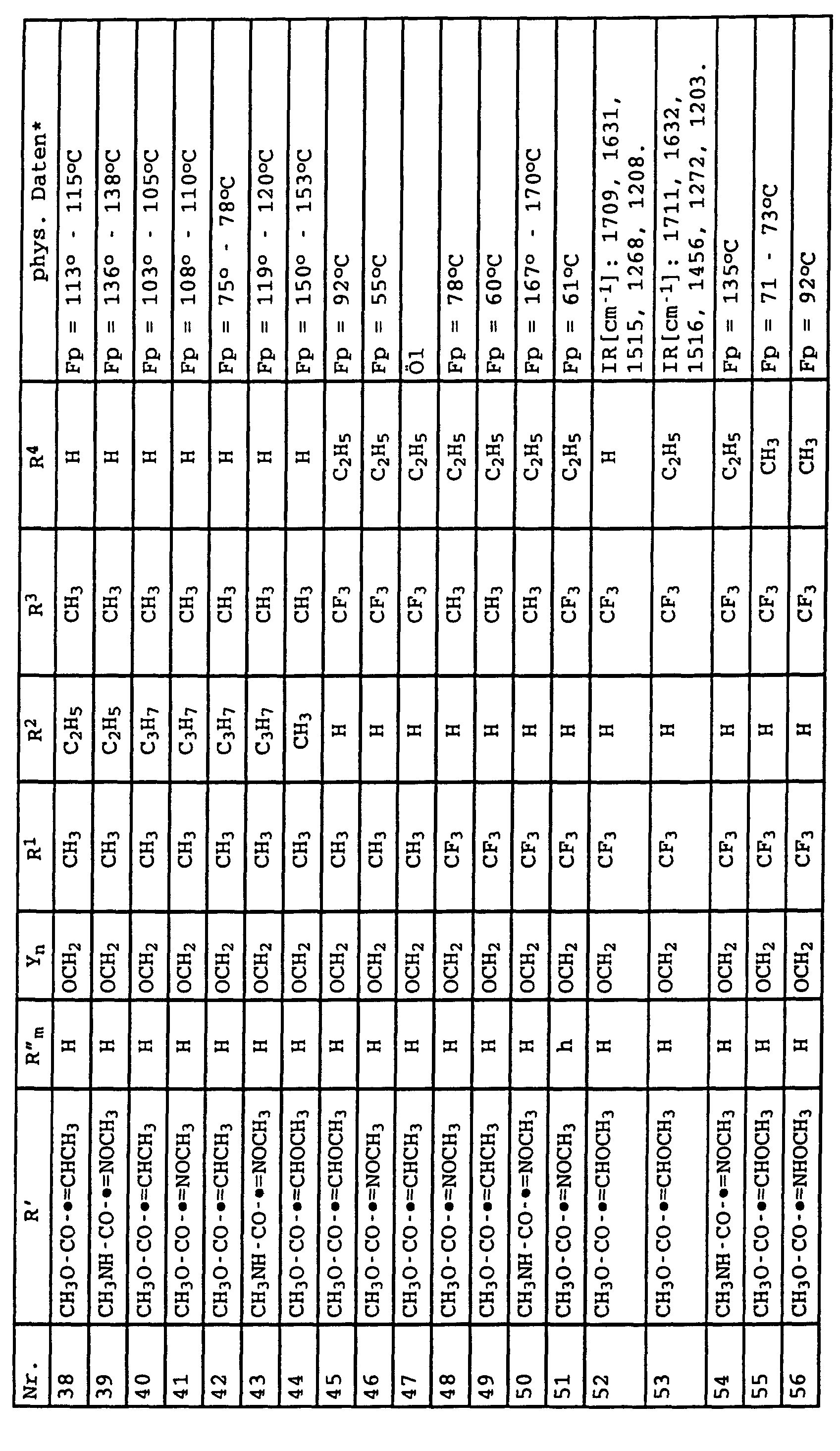

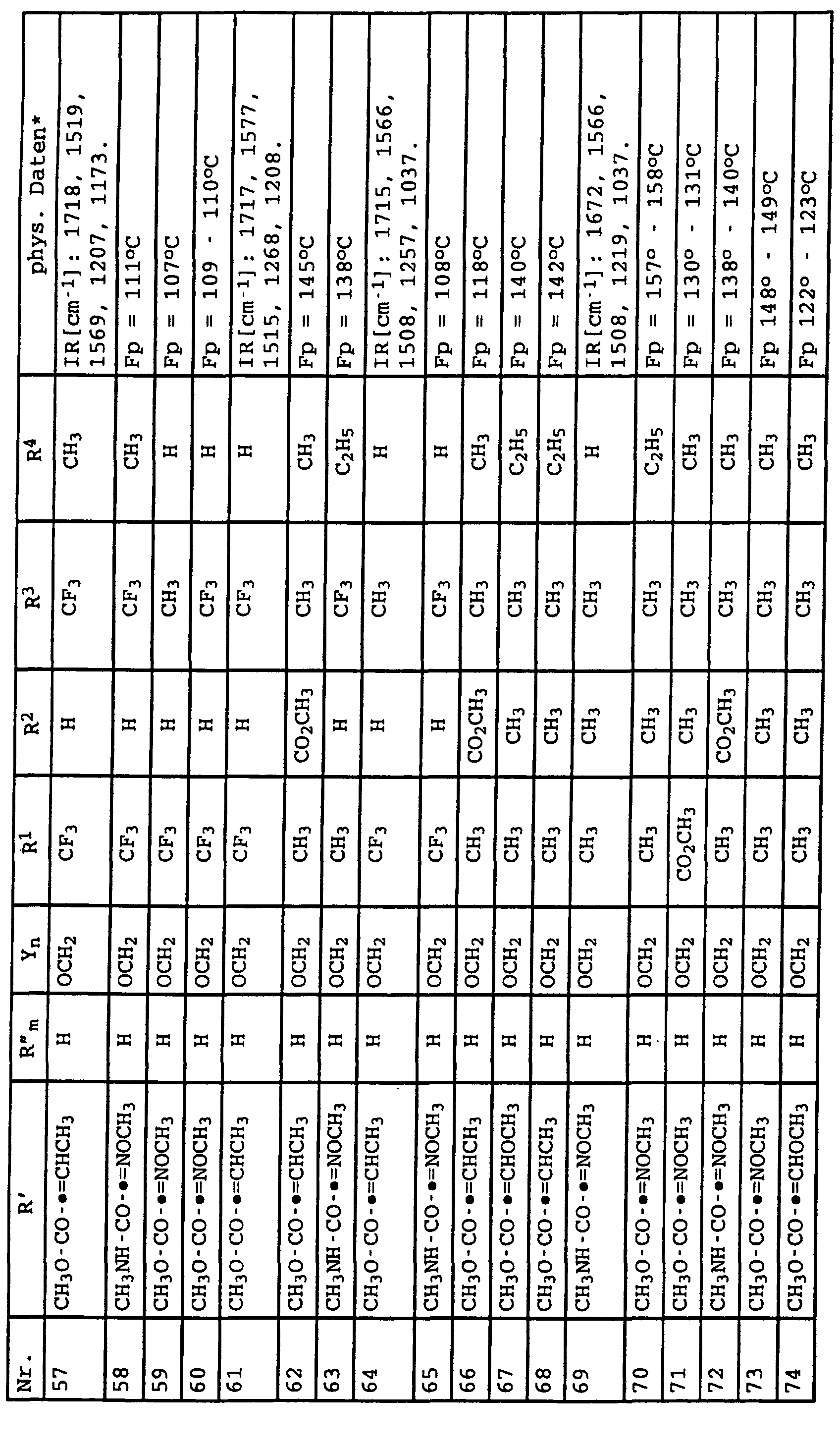

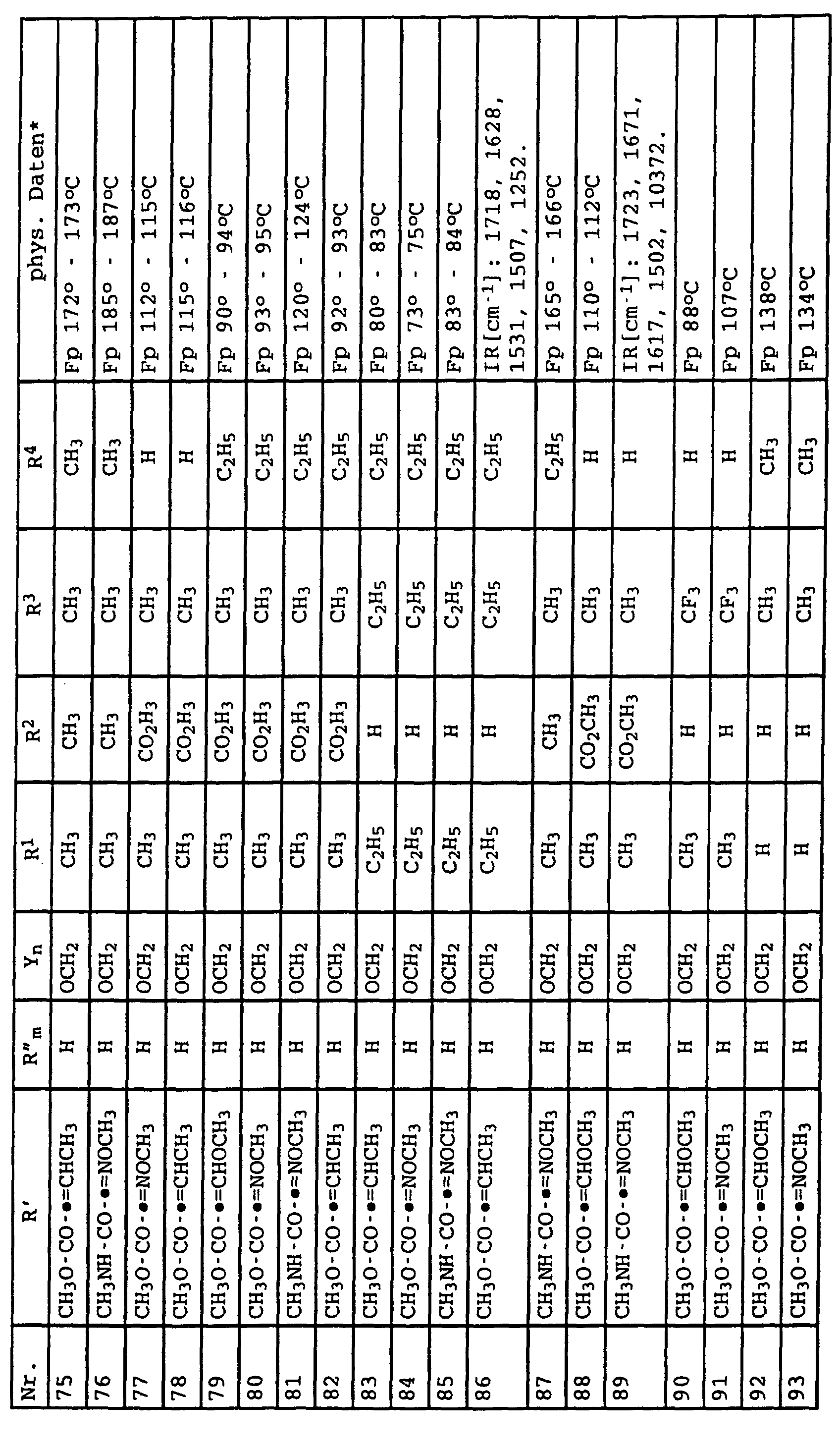

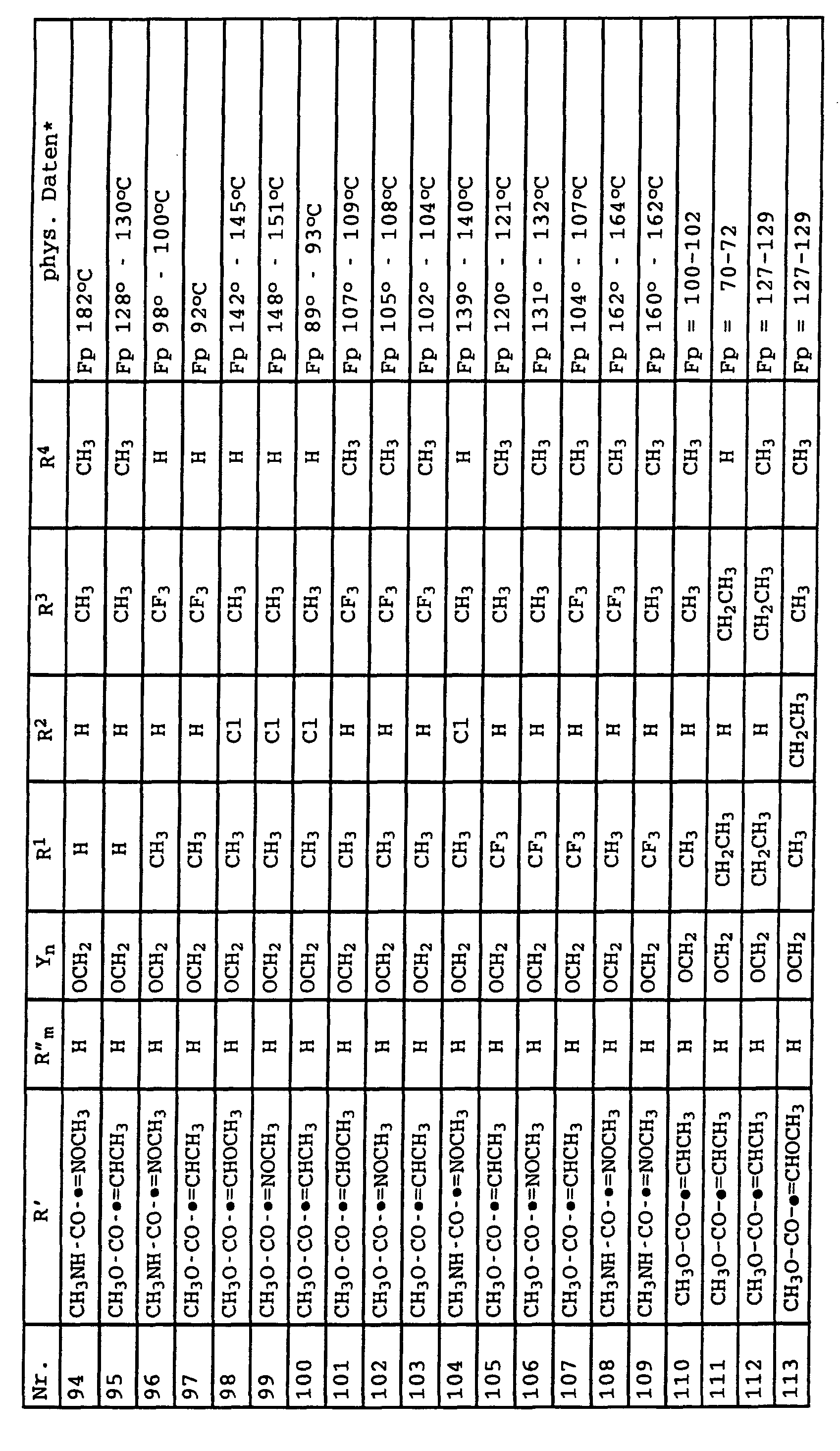

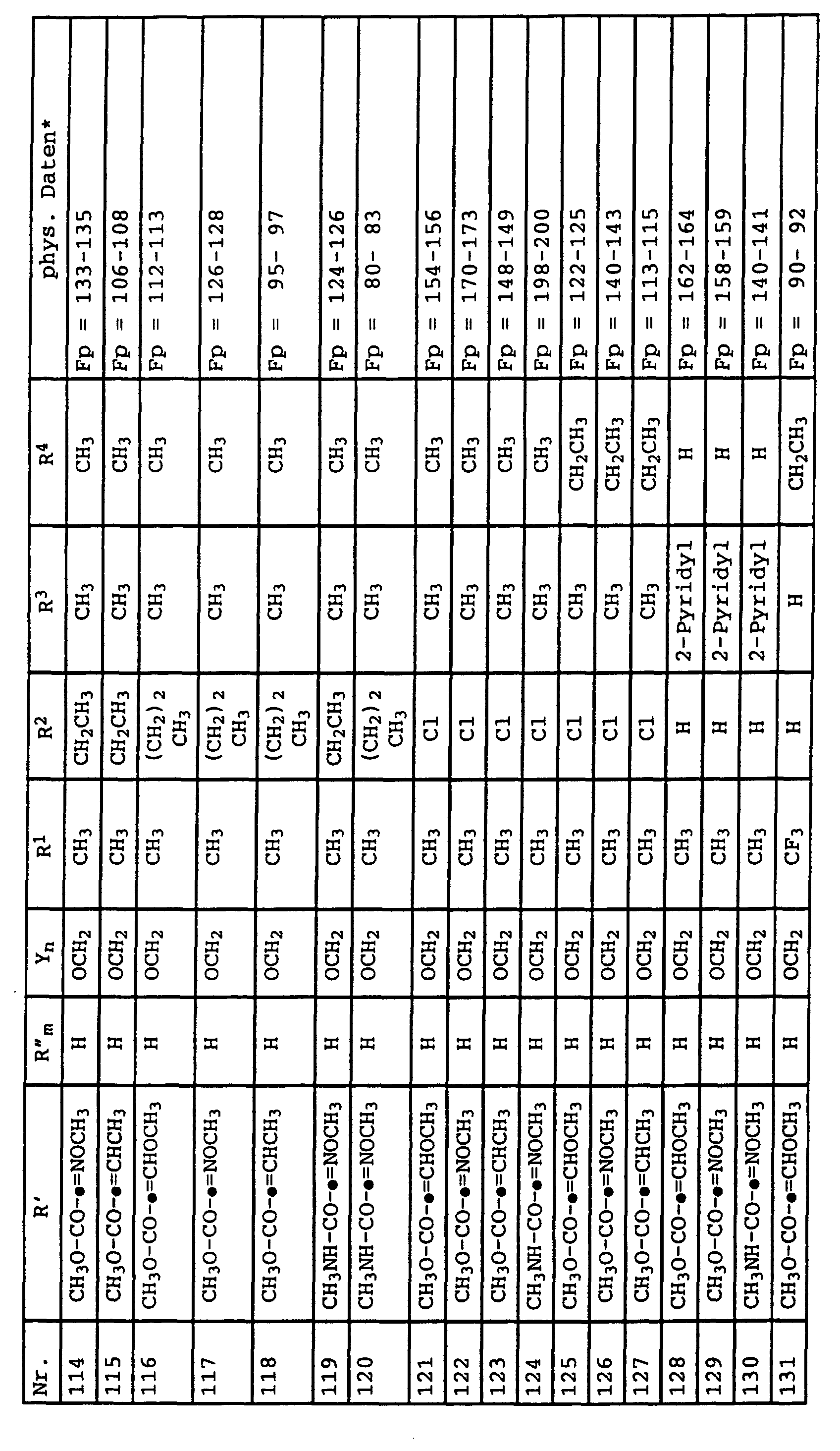

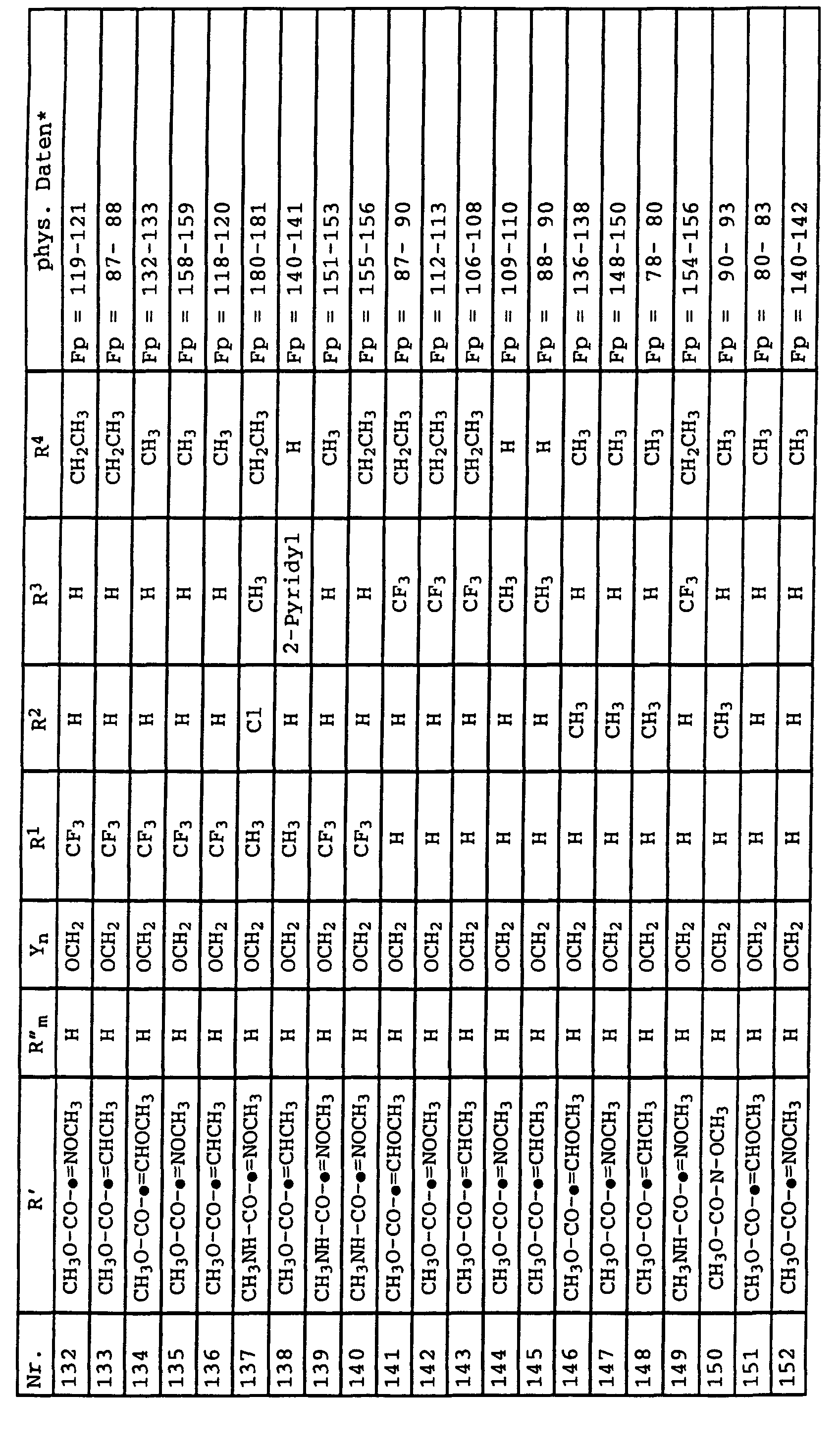

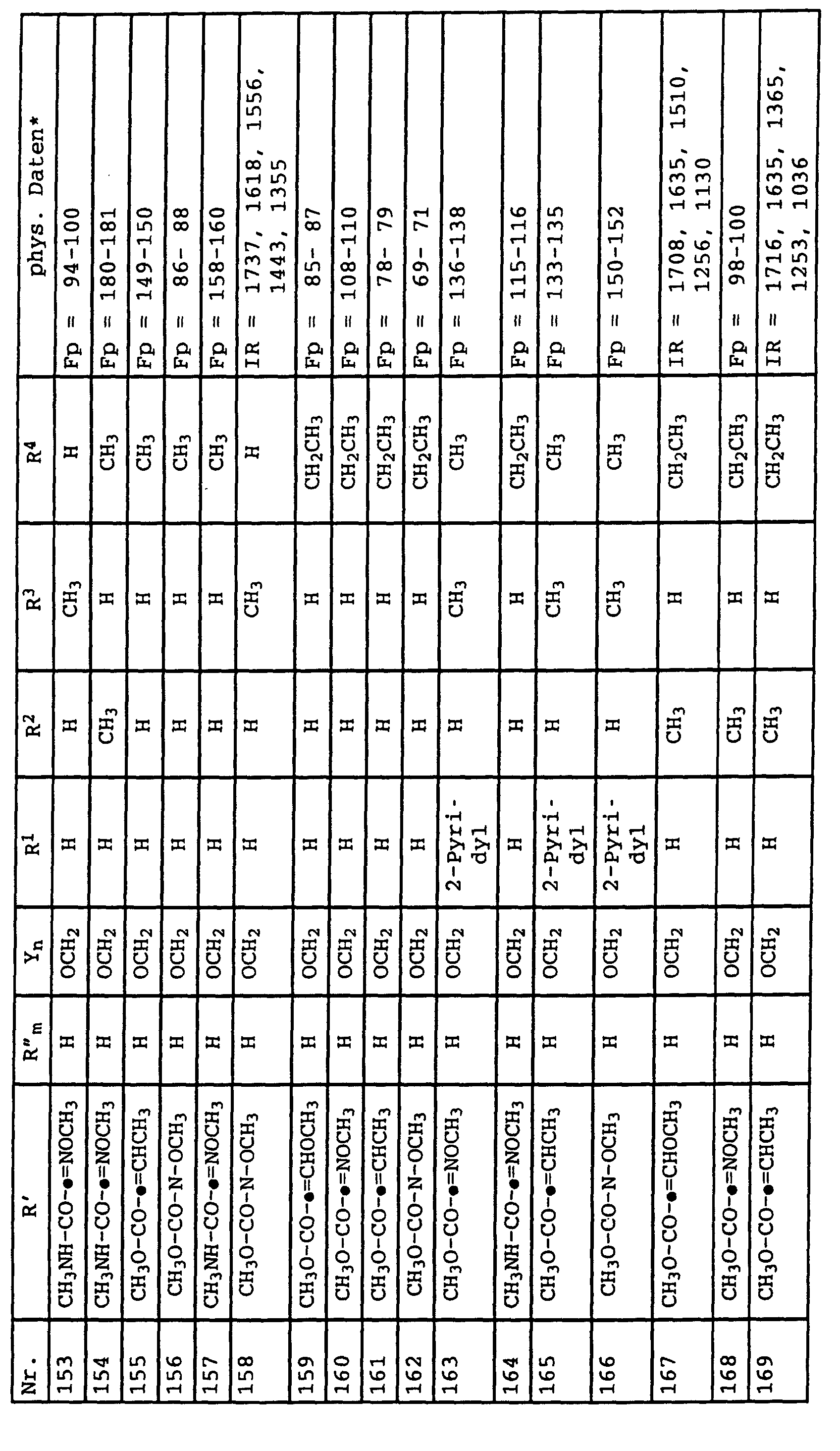

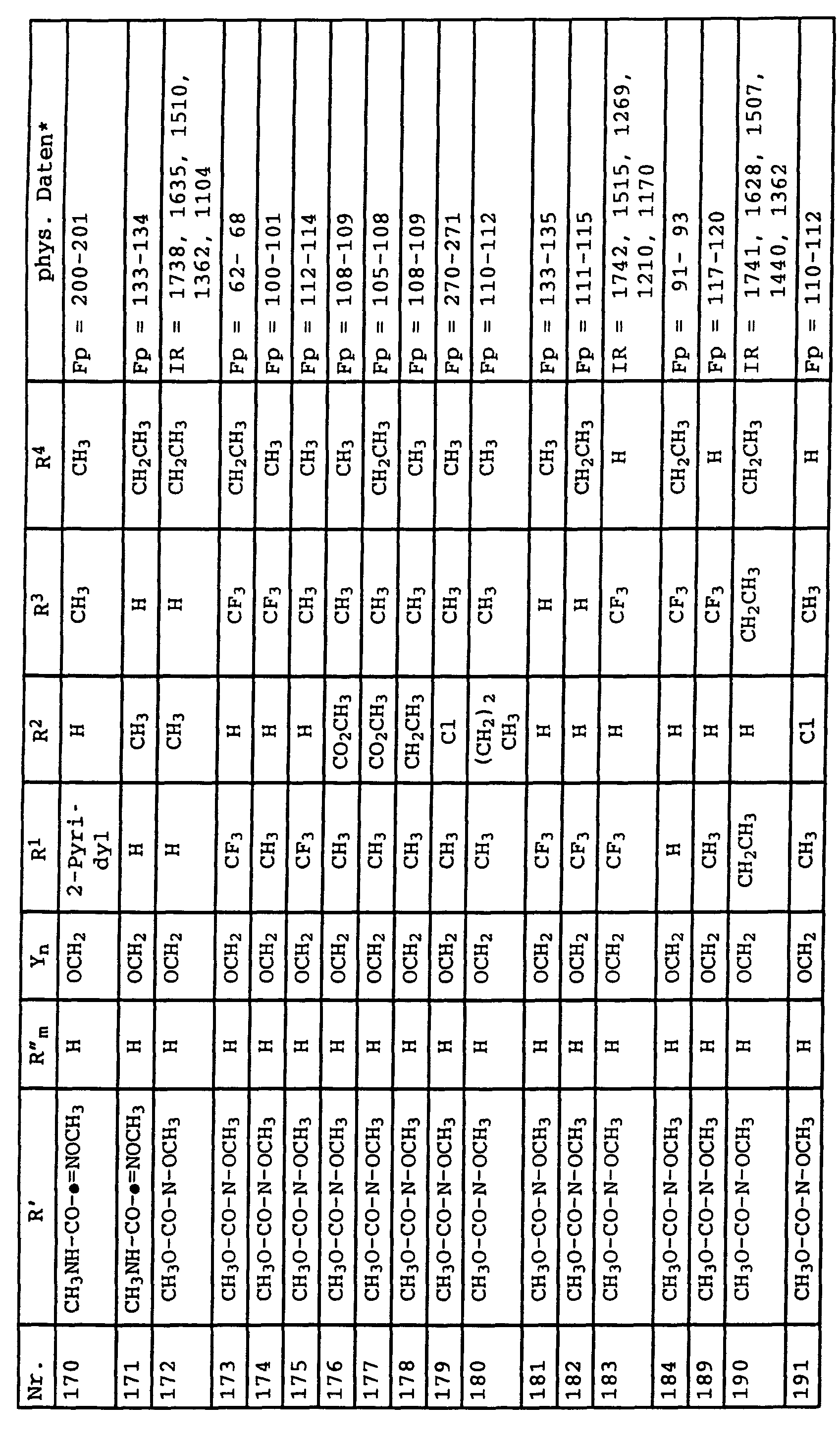

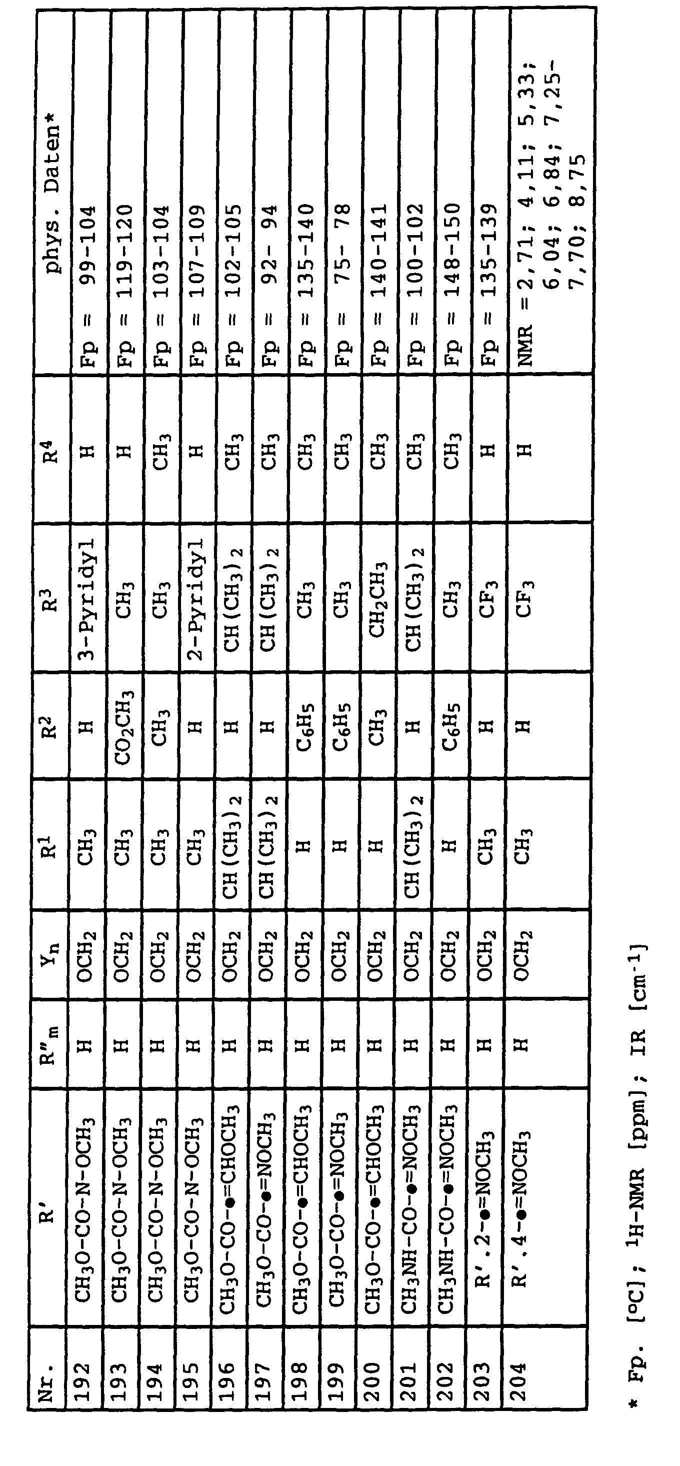

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Puccinia recondita (Weizenbraunrost)

Blätter von Weizensämlingen (Sorte "Kanzler") wurden mit Sporen des Braunrosts (*Puccinia recondita*) bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung (Aufwandmenge: 250 ppm) behandelt. Nach weiteren 8 Tagen bei 20-22°C und 65-70% relativer Luftfeuchtigkeit wurde das Ausmaß der Pilzentwicklung ermittlelt. Die Auswertung erfolgte visuell.

Bei diesem Test zeigten die mit den erfindungsgemäßen Verbindungen 2, 3, 7, 11, 12, 14, 17, 19, 21-23, 26, 30, 34-36, 39, 40 43, 47, 49, 52, 53, 55, 57, 58 und 62 behandelten Pflanzen einen Befall von 15% und weniger während die unbehandelten (Kontroll-) Pflanzen zu 70% befallen waren.

In einem entsprechenden Test zeigten die mit 250 ppm der Verbindungen 64, 66, 68, 69, 72, 74, 78, 79, 81, 82, 83, 85, 86, 88, 89, 90, 92, 94, 96, 97, 98, 100, 101, 102, 103, 104, 105, 107, 108, 109, 110, 111, 113, 116, 118, 119, 120, 121, 125, 127, 131, 166, 167, 175, 176, 177, 178, 180, 190, 193, 194, 196, 198, 201, 202 und 204 behandelten Pflanzen einen Befall von 15% und weniger, während die unbehandelten (Kontroll-) Pflanzen zu 70% befallen waren.

### Wirkung gegen Pyricularia oryzae (Reisbrand)

Reiskeimlinge (Sorte: "Tai Nong 67") wurden mit der Wirkstoffaufbereitung (Aufwandmenge: 63 ppm) tropfnaß gespritzt. Nach 24 Stunden wurden die Pflanzen mit einer wäßrigen Sporensuspension des Pilzes *Pyricularia oryzae* besprüht und 6 Tage bei 22-24 °C bei einer relativen Luftfeuchtigkeit von 95-99 % bewahrt.

Die Beurteilung erfolgte visuell.

Bei diesem Test zeigten die mit den erfindungsgemäßen Verbindungen 2, 3, 6, 7, 11, 17, 21-23, 30, 35, 36, 40, 43, 47-49, 52, 53, 55-58 und 62 behandelten Pflanzen einen Befall von 15% und weniger während die unbehandelten (Kontroll-) Pflanzen zu 65% befallen waren.

In einem entsprechenden Test zeigten die mit 63 ppm der Verbindungen 66, 67, 71, 72, 73, 76, 77, 78, 79, 80, 81, 82, 86, 88, 89, 90, 91, 92, 94, 95, 96, 97, 98, 100, 101, 102, 103, 104, 105, 107, 109, 110, 111, 116, 118, 119, 125, 127,129,130, 131, 134, 135, 137, 139, 174, 175, 176 und 178 behandelten Pflanzen einen Befall von 15% und weniger, während die unbehandelten (Kontroll-) Pflanzen zu 85% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

### Aphis fabae (Schwarze Laus), Kontaktwirkung

Stark befallene Buschbohnen (Vicia faba) wurden mit der wässrigen Wirkstoffaufbereitung behandelt. Nach 24h wurde die Mortalitätsrate bestimmt.

In diesem Test zeigten die Verbindungen 25, 35, 141, 193, 196 und 197 Wirkschwellen von 400 ppm und weniger.

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigten die Verbindungen 53, 83, 125, 141, 193, 196, 197 und 201 Wirkschwellen von 0,4 mg und weniger.

### Prodenia litura (Ägyptischer Baumwollwurm), Kontaktwirkung

Mit der wäßrigen Wirkstoffaufbereitung behandelte Filter wurden mit 5 Raupen belegt. Die erste Beurteilung erfolgt nach 4h. Sofern noch mindestens eine Raupe lebt, wird eine Futtermischung zugegeben. Nach 24h wurde die Mortalität bestimmt.

In diesem Test zeigten die Verbindungen 45, 46, 55, 141, 196 und 197 Wirkschwellen von 0,4 mg und weniger.

### Heliothis virescens (Baumwolleule), Kontakt-/Fraßwirkung

Ca. 10 cm große Tabakpflanzen wurden mit der wäßrigen Wirkstoffaufbereitung behandelt. Nach dem Abtrocknen wurden die Pflanzen mit jeweils 10 Larven des 3. Enwicklungsstadiums belegt. Nach 48h wurden Mortalität und Fraßverhinderung beurteilt.

In diesem Test zeigten die Verbindungen 55, 196 und 197 Wirkschwellen von 400 ppm und weniger.

### Tetranychus telarius (Rote Spinne), Kontaktwirkung

Stark befallene getopfte Buschbohnen, die das zweite Folgeblattpaar zeigten, wurden mit wässrigen Wirkstoffaufbereitung behandelt. Nach 5 Tagen im Gewächshaus wurde der Bekämpfungserfolg mittels Binokular bestimmt.

In diesem Test zeigten die Verbindungen 17, 25, 35, 46, 47, 49, 51, 51, 52, 54, 57, 63, 66, 79, 80, 82, 83, 84, 85, 86, 125, 127, 131, 141, 150, 151, 169, 173, 196, 197, 198, 199 und 201 Wirkschwellen von 400 ppm und weniger.

## Patentansprüche

1. Pyrazolo-[1,5a]-pyrimidine der Formel I 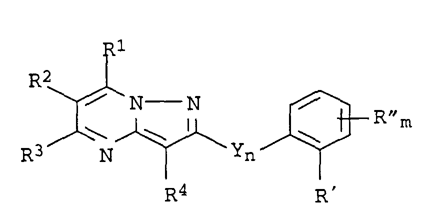 in der die Indizes und die Substituenten die folgende Bedeutung haben:
n 0 oder 1;
Y O, S, NR^{a}, OCH₂*, SCH₂*, NR^{a}CH₂*, CH₂O*, CH₂S*, CH₂NR^{a}*, CH₂CH₂, CR^{a}=CR^{b} oder C≡C, wobei die mit * gekennzeichneten Positionen die Bindung zum Phenylring kennzeichnen;
R' H₃CO-CO-•=CHOCH₃, H₃CNH-CO-•=CHOCH₃, H₃CO-CO-•=NOCH₃, H₃CNH-CO-•=NOCH₃, H₂N-CO-•=NOCH₃, HO-CO-•=NOCH₃, H₃CO-CO-•=CHCH₃, H₃CO-CO-•=CHCH₂CH₃, H₃C-CO-•=COHCH₃, H₃C-CO-•=NOCH₃, H₃CCH₂-CO-•=NOCH₃, wobei • ein zum Phenylring gebundenes C-Atom darstellt;
N(OCH₃)-CO₂CH₃, N(CH₃)-CO₂CH₃, N(CH₂CH₃)-CO₂CH₃, oder
eine Gruppe R'.1-•=NOCH₃, R'.2-•=NOCH₃, R'.3-•=NOCH₃ oder R'.4-•=NOCH₃, 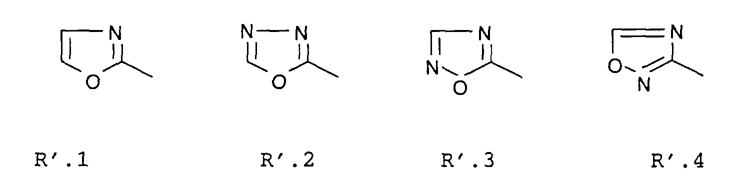
m 0, 1, 2 oder 3, wobei die Reste R" verschieden sein können, wenn m größer als 1 ist;
R" Cyano, Halogen, C₁-C₄-Alkyl, C₁-C₄-Haloalkyl, C₁-C₄-Alkoxy, C₁-C₄-Haloalkoxy, C₁-C₄-Alkylthio oder C₁-C₄-Haloalkylthio;
R¹,R³ Wasserstoff, Cyano, Nitro, Hydroxy, Amino, Halogen,
Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Phenyl, Phenyl-C₁-C₄-alkyl, Phenoxy, Phenyl-C₁-C₄-alkoxy, Phenylthio oder
NR^{c}R^{d}, NR^{c}NR^{d}R^{e}, CO-R^{c}, CO₂-R^{c}, CO-NR^{c}R^{d}, O-CO-R^{c}, O-CO₂-R^{c}, O-CO-NR^{c}R^{d}, NR^{c}-CO-R^{d}, NR^{c}-CO₂-R^{d} oder NR^{c}-CO-NR^{d}R^{e};
R² C₁-C₆-Wasserstoff, Halogen, C₁-C₆-Alkyl, C₁-C₆-Halogenalkyl, C₁-C₆-Alkoxy, C₁-C₆-Halogenalkoxy, Phenyl, Phenoxy, CO-R^{c}, CO₂R^{c} oder CR^{f}=NOR^{g};
R⁴ Wasserstoff, Cyano, Nitro, Nitroso, Halogen,
C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Phenyl, Phenyl-C₁-C₄-alkyl, Phenoxy, Phenyl-C₁-C₄-alkoxy, Phenylthio, oder NR^{c}R^{d}, CO-R^{c}, CO₂-R^{c} oder CR^{f}=NOR^{g};
R^{a}, R^{b} Wasserstoff, Halogen, C₁-C₄-Alkyl oder C₁-C₄-Halogenalkyl;
R^{c}, R^{d} und R^{e}
Wasserstoff, C₁-C₆-Alkyl, C₃-C₆-Alkenyl, C₃-C₆-Alkinyl, Phenyl oder Phenyl-C₁-C₄-alkyl;
R^{f} Wasserstoff, Cyano, Halogen,
C₁-C₆-Alkyl, C₁-C₆-Alkyloxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy, C₃-C₆-Alkinylthio, C₃-C₆-Cycloalkyl,
R^{g} Wasserstoff,
C₁-C₁₀-Alkyl, C₃-C₁₀-Alkenyl, C₃-C₁₀-Alkinyl, C₃-C₆-Cycloalkyl oder Phenyl.

2. Pyrazolo-[1,5a]-pyrimidine der Formel I gemäß Anspruch 1, in der die Substituenten R¹ bis R⁴ die folgende Bedeutung haben:
Wasserstoff, C₁-C₄-Alkyl, C₁-C₄-Halogenalkyl, C₁-C₄-Alkoxy, C₁-C₄-Alkoxycarbonyl oder Phenyl.

3. Verfahren zur Herstellung der Verbindungen I, in denen Yₙ für OCH₂*, SCH₂* oder NR^{a}CH₂* [kollektiv als *"XCH*_{*2*}*"* bezeichnet] steht, **dadurch gekennzeichnet, daß** man ein Benzylderivat der Formel IIa 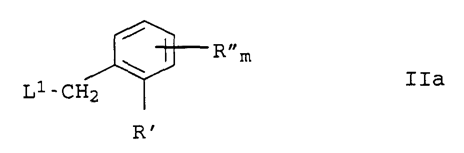 in der L¹ für eine nucleophil austauschbare Abgangsgruppe steht, in an sich bekannter Weise in einem inerten organischen Lösungsmittel in Gegenwart einer Base mit einem Pyrazolopyrimidin der Formel IIIa 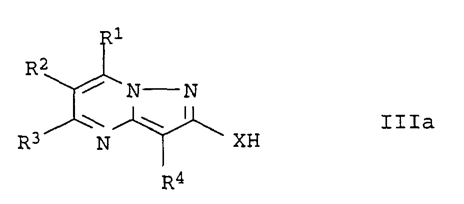 umsetzt.

4. Verfahren zur Herstellung der Verbindungen I, in denen Yₙ für C=C steht, **dadurch gekennzeichnet, daß** man ein Phenylacetylen der Formel IIb 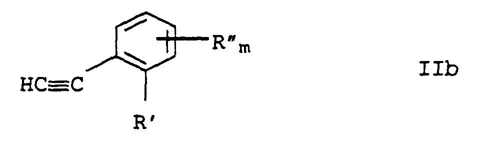 in an sich bekannter Weise in einem inerten Lösungsmittel in Gegenwart eines Edelmatallkatalysators mit ein Pyrazolopyrimidinylhalogenid der Formel IIIb 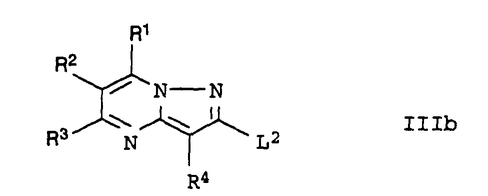 in der L² für ein Halogenatom steht, umsetzt.

5. Verfahren zur Herstellung der Verbindungen I, in denen Yₙ für CH₂O*, CH₂S* oder CH₂NR^{a}* [kollektiv als *"CH*_{*2*}*X"* bezeichnet] steht, **dadurch gekennzeichnet, daß** man ein entsprechendes Phenylderivat IIc 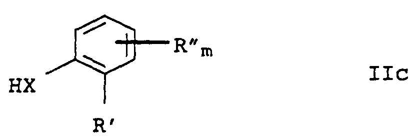 in an sich bekannter Weise in einem inerten Lösungsmittel in Gegenwart einer Base mit einem Pyrazolopyrimidinylmethylenhalogenid der Formel IIIc 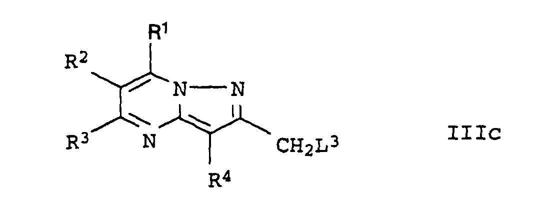 in der L³ für eine nucleophil austauschbare Abgangsgruppe steht, umsetzt.

6. Verfahren zur Herstellung der Verbindungen I gemäß Anspruch 1, in denen Yₙ für O, S oder NR⁵ [kollektiv als *"X"* bezeichnet] steht, **dadurch gekennzeichnet, daß** man ein entsprechendes Phenylderivat der Formel IIc gemäß Anspruch 5 in an sich bekannter Weis ein einem inerten Lösungsmittel in Gegenwart einer Base mit einem Pyrazolopyrimidinylhalogenid der Formel IIIb gemäß Anspruch 4 kuppelt.

7. Verfahren zur Herstellung der Verbindungen I gemäß Anspruch 1, in denen Yₙ für CR^{a}=CR^{b} steht, **dadurch gekennzeichnet, daß** man eine Benzylphoshorverbindung der allgemeinen Formel IId 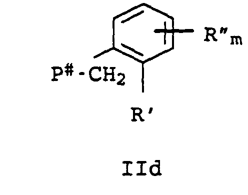 in der P# für eine Triarylohosphonium-halogenid- oder Dialkoxyphosphonat-Gruppierung steht, im Sinne einer Wittig- oder Horner-Wittig Umsetzung mit einem Benzaldehyd der Formel IIId 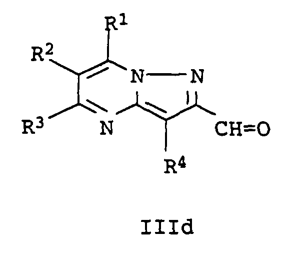 in I überführt.

8. Verfahren zur Herstellung der Verbindungen I gemäß Anspruch 1, in denen Yₙ für CR^{a}=CR^{b} steht, **dadurch gekennzeichnet, daß** man eine Benzylphoshorverbindung IIId 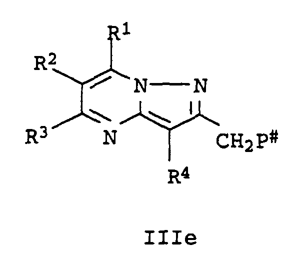 in der P# für eine Triarylohosphonium-halogenid- oder Dialkoxyphosphonat-Gruppierung steht, im Sinne einer Wittig- oder Horner-Wittig Umsetzung mit einem Benzaldehyd der Formel IIe 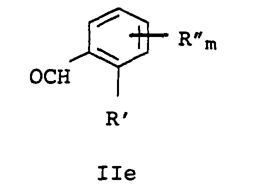 in I überführt.

9. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

10. Verwendung der Verbindungen I gemäß Anspruch 1 zur Herstellung eines zur Bekämpfung von Schädlingen oder Schadpilzen geeigneten Mittels.

11. Verfahren zur Bekämpfung von Schadpilzen, **dadurch gekennzeichnet, daß** man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

12. Verfahren zur Bekämpfung von Schädlingen, **dadurch gekennzeichnet, daß** man die Schädlinge oder die vor ihnen zu schützenden Materialien, Pflanzen, Boden oder Saatgüter mit einer wirksamen Menge einer Verbindung der allgemeinen Formel I gemäß Anspruch 1 behandelt.

## Claims

1. A pyrazolo[1,5a]pyrimidine of the formula I 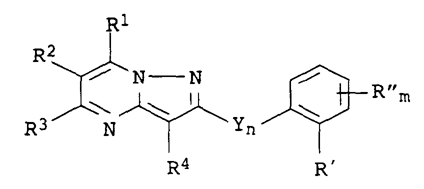 where the indices and the substituents have the following meanings:
n is 0 or 1;
Y is O, S, NR^{a}, OCH₂*, SCH₂*, NR^{a}CH₂*, CH₂O*, CH₂S*, CH₂NR^{a}*, CH₂CH₂, CR^{a}=CR^{b} or C≡C, the positions identified by * marking the bond to the phenyl ring;
R' is H₃CO-CO-•=CHOCH₃, H₃CNH-CO-•=CHOCH₃, H₃CO-CO-•=NOCH₃, H₃CNH-CO-•=NOCH₃, H₂N-CO-•=NOCH₃, HO-CO-•=NOCH₃, H₃CO-CO-•=CHCH₃, H₃CO-CO-•=CHCH₂CH₃, H₃CO-CO-•=COHCH₃, H₃C-CO-•=NOCH₃, H₃CCH₂-CO-•=NOCH₃, • being a C atom bonded to the phenyl ring;
N(OCH₃)-CO₂CH₃, N(CH₃)-CO₂CH₃, N(CH₂CH₃)-CO₂CH₃, or
a group R'.1-•=NOCH₃, R'.2-•=NOCH₃, R'.3-•=NOCH₃ or R'.4-•=NOCH₃, 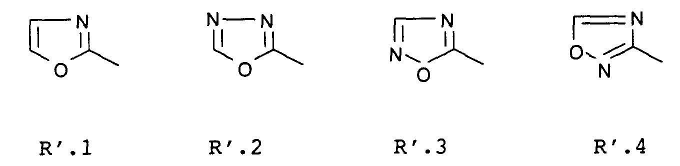
m is 0, 1, 2 or 3, it being possible for radicals R" to be different if m is greater than 1;
R" is cyano, halogen, C₁-C₄-alkyl, C₁-C₄-haloalkyl, C₁-C₄-alkoxy, C₁-C₄-haloalkoxy, C₁-C₄-alkylthio or C₁-C₄-haloalkylthio;
R¹ and R³ are hydrogen, cyano, nitro, hydroxyl, amino, halogen,
alkyl, C₁-C₆-haloalkyl, C₁-C₆-alkoxy, C₁-C₆-haloalkoxy, C₁-C₆-alkylthio, C₁-C₆-haloalkylthio, phenyl, phenyl-C₁-C₄-alkyl, phenoxy, phenyl-C₁-C₄-alkoxy, phenylthio,
NR^{c}R^{d}, NR^{c}NR^{d}R^{e}, CO-R^{c}, CO₂-R^{c}, CO-NR^{c}R^{d}, O-CO-R^{c}, O-CO₂-R^{c}, O-CO-NR^{c}R^{d}, NR^{c}-CO-R^{d}, NR^{c}-CO₂-R^{d} or NR^{c}-CO-NR^{d}R^{e};
R² is C₁-C₆-hydrogen, halogen, C₁-C₆-alkyl, C₁-C₆-haloalkyl, C₁-C₆-alkoxy, C₁-C₆-haloalkoxy, phenyl, phenoxy, CO-R^{c}, CO₂R^{c} or CR^{f}=NOR^{g};
R⁴ is hydrogen, cyano, nitro, nitroso, halogen,
C₁-C₆-alkyl, C₁-C₆-haloalkyl, C₁-C₆-alkoxy, C₁-C₆-haloalkoxy, C₁-C₆-alkylthio, C₁-C₆-haloalkylthio, phenyl, phenyl-C₁-C₄-alkyl, phenoxy, phenyl-C₁-C₄-alkoxy, phenylthio, or NR^{c}R^{d}, CO-R^{c}, CO₂-R^{c} or CR^{f}=NOR^{g};
R^{a} and R^{b} are hydrogen, halogen, C₁-C₄-alkyl or C₁-C₄-haloalkyl;
R^{c}, R^{d} and R^{e}
are hydrogen, C₁-C₆-alkyl, C₃-C₆-alkenyl, C₃-C₆-alkynyl, phenyl or phenyl-C₁-C₄-alkyl;
R^{f} is hydrogen, cyano, halogen,
C₁-C₆-alkyl, C₁-C₆-alkyloxy, C₁-C₆-alkylthio, C₃-C₆-alkenyl, C₃-C₆-alkenyloxy, C₃-C₆-alkenylthio, C₃-C₆-alkynyl, C₃-C₆-alkynyloxy, C₃-C₆-alkynylthio, C₃-C₆-cycloalkyl,
R^{g} is hydrogen,
C₁-C₁₀-alkyl, C₃-C₁₀-alkenyl, C₃-C₁₀-alkynyl, C₃-C₆-cycloalkyl or phenyl.

2. A pyrazolo[1,5a]pyrimidine of the formula I as claimed in claim 1, where the substituents R¹ to R⁴ have the following meanings:
hydrogen, C₁-C₄-alkyl, C₁-C₄-haloalkyl, C₁-C₄-alkoxy, C₁-C₄-alkoxycarbonyl or phenyl.

3. A process for preparing the compounds I where Yₙ is OCH₂*, SCH₂* or NR^{a}CH₂* [collectively designated as *XCH*_{*2*}], which comprises reacting a benzyl derivative of the formula IIa 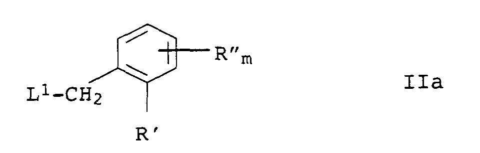 where L¹ is a nucleophilically replaceable leaving group, in a manner known per se in an inert organic solvent in the presence of a base with a pyrazolopyrimidine of the formula IIIa

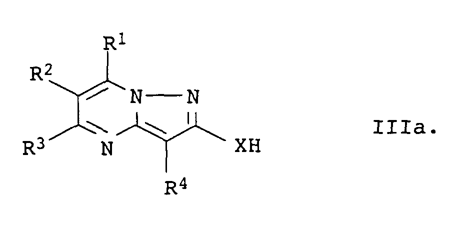

4. A process for preparing the compounds I where Yₙ is C≡C, which comprises reacting a phenylacetylene of the formula IIb 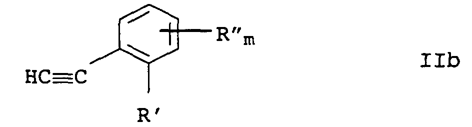 in a manner known per se in an inert solvent in the presence of a noble metal catalyst with a pyrazolopyrimidinyl halide of the formula IIIb 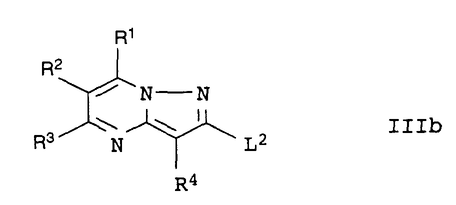 where L² is a halogen atom.

5. A process for preparing the compounds I where Yₙ is CH₂O*, CH₂S* or CH₂NR^{a}* [collectively designated as *CH*_{*2*}*X*], which comprises reacting an appropriate phenyl derivative IIc 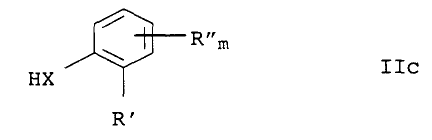 in a manner known per se in an inert solvent in the presence of a base with a pyrazolopyrimidinylmethylene halide of the formula IIIc 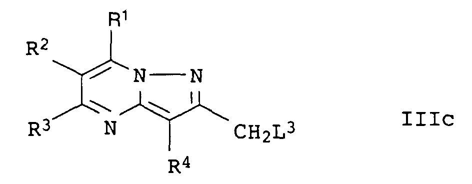 where L³ is a nucleophilically replaceable leaving group.

6. A process for preparing the compounds I as claimed in claim 1, where Yₙ is O, S or NR⁵ [collectively designated as *X*], which comprises coupling an appropriate phenyl derivative of the formula IIc as set forth in claim 5 in a manner known per se in an inert solvent in the presence of a base with a pyrazolopyrimidinyl halide of the formula IIIb as set forth in claim 4.

7. A process for preparing the compounds I as claimed in claim 1, where Yₙ is CR^{a}=CR^{b}, which comprises converting a benzylphosphorus compound of the general formula IId 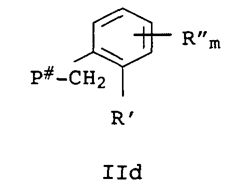 where P# is a triarylphosphonium halide or dialkoxyphosphonate group, according to a Wittig or Wittig-Horner reaction with a benzaldehyde of the formula IIId 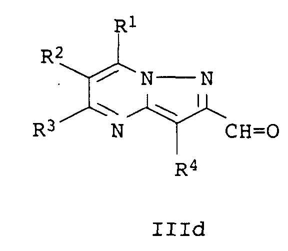 into I.

8. A process for preparing the compounds I as claimed in claim 1, where Yₙ is CR^{a}=CR^{b}, which comprises converting a benzylphosphorus compound IIIe 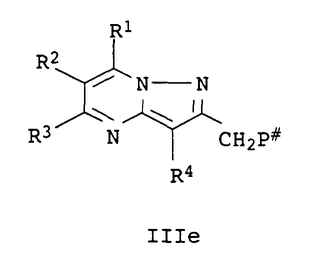 where P# is a triarylphosphonium halide or dialkoxyphosphonate group, according to a Wittig or Wittig-Horner reaction with a benzaldehyde of the formula IIe 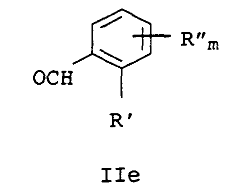 into I.

9. A composition suitable for controlling pests or harmful fungi, containing a solid or liquid carrier and a compound of the general formula I as claimed in claim 1.

10. The use of the compounds I as claimed in claim 1 for preparing a composition suitable for controlling pests or harmful fungi.

11. A method of controlling harmful fungi, which comprises treating the fungi or the materials, plants, soil or seed to be protected from fungal attack with an effective amount of a compound of the general formula I as claimed in claim 1.

12. A method of controlling pests, which comprises treating the pests or the materials, plants, soil or seed to be protected from them with an effective amount of a compound of the general formula I as claimed in claim 1.

## Revendications

1. Pyrazolo-[1,5a]-pyrimidines de formule I 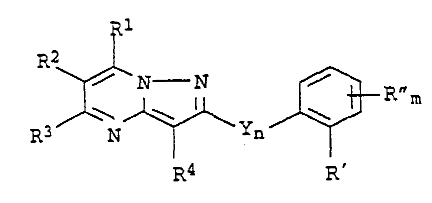 dans laquelle les indices et les symboles ont les significations suivantes :
n : 0 ou 1 ;
Y : O, S, NR^{a}, OCH₂*, SCH₂*, NR^{a}CH₂*, CH₂O*, CH₂S*, CH₂NR^{a}*, CH₂CH₂, CR^{a}=CR^{b} ou C≡C, les positions signalées par un * indiquant la liaison au cycle phényle ;
R' : H₃CO-CO-•=CHOCH₃, H₃CNH-CO-•=CHOCH₃, H₃CO-CO-•=NOCH₃, H₃CNH-CO-•=NOCH₃, H₂N-CO-•=NOCH₃, HO-CO-•=NOCH₃, H₃CO-CO-•=CHCH₃, H₃CO-CO-•=CHCH₂CH₃, H₃C-CO-•=COHCH₃, H₃C-CO-•=NOCH₃, H₃CCH₂-CO-•=NOCH₃, le signe • représentant un atome de carbone relié au cycle phényle ;
N(OCH₃)-CO₂CH₃, N(CH₃)-CO₂CH₃, N(CH₂CH₃)-CO₂CH₃,
ou
un groupe R'.1-•=NOCH₃, R'.2-•=NOCH₃, R'.3-•=NOCH₃ ou R'.4-•=NOCH₃, 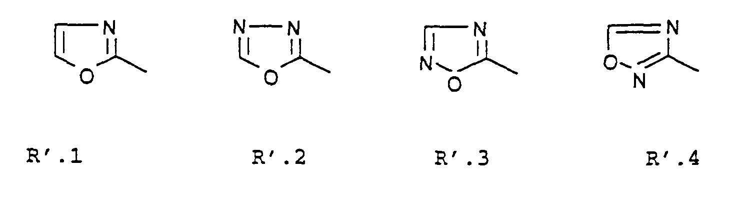
m : 0, 1, 2 ou 3, les substituants R" pouvant être différents lorsque m est supérieur à 1 ;
R" : un groupe cyano, un halogène, un groupe alkyle en C1-C4, halogénoalkyle en C1-C4, alcoxy en C1-C4, halogénoalcoxy en C1-C4, alkylthio en C1-C4 ou halogénoalkylthio en C1-C4 ;
R¹, R³ : l'hydrogène, un groupe cyano, nitro, hydroxy, amino, un halogène,
un groupe alkyle, halogénoalkyle en C1-C6, alcoxy en C1-C6, halogénoalcoxy en C1-C6, alkylthio en C1-C6, halogénoalkylthio en C1-C6, phényle, phényl-alkyle en C1-C4, phénoxy, phényl-alcoxy en C1-C4, phénylthio ou
NR^{c}R^{d}, NR^{c}NR^{d}R^{e}, CO-R^{c}, CO₂-R^{c}, CO-NR^{c}R^{d}, O-CO-R^{c}, O-CO₂-R^{c}, O-CO-NR^{c}R^{d}, NR^{c}-CO-R^{d}, NR^{c}-CO₂-R^{d} ou NR^{c}-CO-NR^{d}R^{e} ;
l'hydrogène, un halogène, un groupe alkyle en C1-C6, halogénoalkyle en C1-C6, alcoxy en C1-C6, halogénoalcoxy en C1-C6, phényle, phénoxy, -CO-R^{c}, CO₂R^{c} ou CR^{f}=NOR^{g} ;
R⁴ : l'hydrogène, un groupe cyano, nitro, nitroso, un halogène, un groupe alkyle en C1-C6, halogénoalkyle en C1-C6, alcoxy en C1-C6, halogénoalcoxy en C1-C6, alkylthio en C1-C6, halogénoalkylthio en C1-C6, phényle, phényl-alkyle en C1-C4, phénoxy, phényl-alcoxy en C1-C4, phénylthio ou
NR^{c}R^{d}, CO-R^{c}, CO₂-R^{c} ou CR^{f}=NOR^{g} ;
R^{a}, R^{b} : l'hydrogène, un halogène, un groupe alkyle en C1-C4 ou halogénoalkyle en C1-C4 ;
R^{c}, R^{d} et R^{e} : l'hydrogène, un groupe alkyle en C1-C6, alcényle en C3-C6, alcynyle en C3-C6, phényle ou phényl-alkyle en C1-C4 ;
R^{f} : l'hydrogène, un groupe cyano, un halogène,
un groupe alkyle en C1-C6, alcoxy en C1-C6, alkylthio en C1-C6, alcényle en C3-C6, alcényloxy en C3-C6, alcénylthio en C3-C6, alcynyle en C3-C6, alcynyloxy en C3-C6, alcynylthio en C3-C6, cycloalkyle en C3-C6,
R^{g} : l'hydrogène,
un groupe alkyle en C1-C10, alcényle en C3-C10, alcynyle en C3-C10, cycloalkyle en C3-C6 ou phényle.

2. Pyrazolo-[1,5a]-pyrimidines de formule I de la revendication 1, dans laquelle les symboles R¹ à R⁴ ont les significations suivantes : l'hydrogène, des groupes alkyle en C1-C4, halogénoalkyle en C1-C4, alcoxy en C1-C4, (alcoxy en C1-C4)carbonyle ou phényle.

3. Procédé de préparation des composés I pour lesquels Yₙ représente OCH₂*, SCH₂* ou NR^{a}CH₂* [représentés collectivement par "XCH₂"], **caractérisé par le fait que** l'on fait réagir un dérivé benzylique de formule IIa 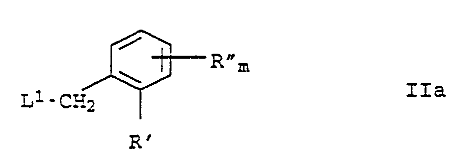 dans laquelle L¹ représente un groupe éliminable par échange nucléophile, de manière connue en soi et dans un solvant organique inerte, en présence d'une base, avec une pyrazolopyrimidine de formule IIIa

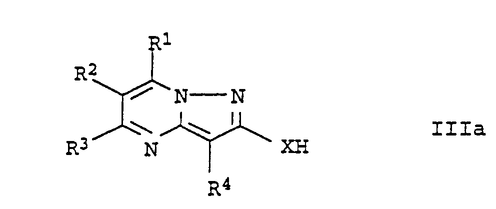

4. Procédé de préparation des composés I pour lesquels Yₙ représente C≡C, **caractérisé par le fait que** l'on fait réagir un phénylacétylène de formule IIb 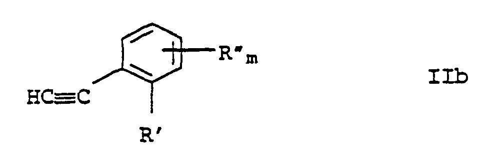 de manière connue en soi, dans un solvant inerte et en présence d'un catalyseur à base d'un métal noble, avec un halogénure de pyrazolopyrimidinyle de formule IIIb 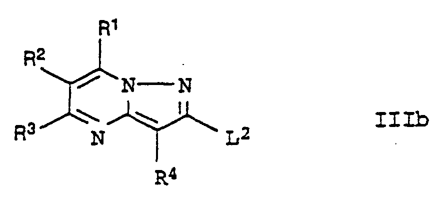 dans laquelle L² représente un atome d'halogène.

5. Procédé de préparation des composés I pour lesquels Yₙ représente CH₂O*, CH₂S* ou CH₂NR^{a}* [représentés collectivement par "CH₂X"] **caractérisé par le fait que** l'on fait réagir un dérivé phénylique correspondant IIc 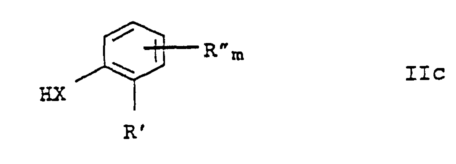 de manière connue en soi, dans un solvant inerte et en présence d'une base, avec un halogénure de pyrazolopyrimidinylméthylène de formule IIIc 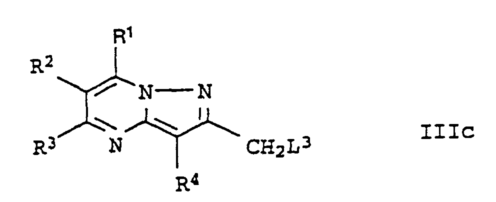 dans laquelle L³ représente un groupe éliminable par échange nucléophile.

6. Procédé de préparation des composés I de la revendication 1, pour lesquels Yₙ représente O, S ou NR⁵ [représentés collectivement par "X"] **caractérisé par le fait que** l'on condense un dérivé phénylique correspondant de formule IIc de la revendication 5, de manière connue en soi, dans un solvant inerte et en présence d'une base, avec un halogénure de pyrazolopyrimidinyle de formule IIIb de la revendication 4.

7. Procédé de préparation des composés I de la revendication 1 pour lesquels Yₙ représente CR^{a}=CR^{b}, **caractérisé par le fait que** l'on convertit un dérivé benzylphosphoré de formule générale IId 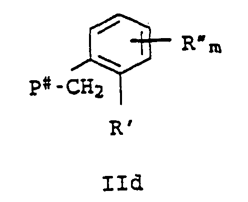 dans laquelle P# représente un groupement halogénure de triarylphosphonium ou dialcoxyphosphonate, par une réaction de Wittig ou d'Horner-Wittig avec un benzaldéhyde de formule IIId 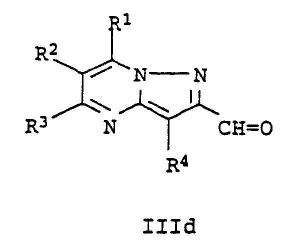 en I.

8. Procédé de préparation des composés I de la revendication 1, pour lesquels Yₙ représente CR^{a}=CR^{b}, **caractérisé par le fait que** l'on convertit un dérivé benzylphosphoré IIId 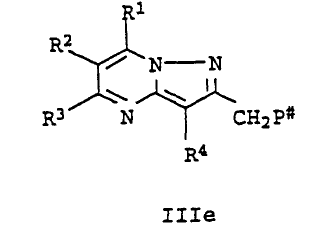 dans laquelle P# représente un groupement halogénure de triarylphosphonium ou dialcoxyphosphonate, par une réaction de Wittig ou d'Horner-Wittig avec un benzaldéhyde de formule IIe 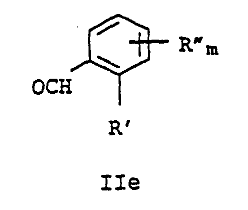 en I.

9. Produit approprié à l'utilisation pour la lutte contre les parasites ou les mycètes nuisibles, contenant un véhicule solide ou liquide et un composé de formule générale I de la revendication 1.

10. Utilisation des composés I de la revendication 1 pour la préparation d'un produit approprié à l'utilisation pour la lutte contre les parasites ou les mycètes nuisibles.

11. Procédé pour combattre les mycètes nuisibles, **caractérisé par le fait que** l'on traite les mycètes ou les matériaux, végétaux, sols ou semences qu'on veut protéger contre une attaque par les mycètes par une quantité efficace d'un composé de formule générale I de la revendication 1.

12. Procédé pour combattre les parasites, **caractérisé par le fait que** l'on traite les parasites ou les matériaux, végétaux, sols ou semences qu'on veut protéger contre les parasites par une quantité efficace d'un composé de formule générale I de la revendication 1.
